# EUROPEAN PATENT APPLICATION

(11) **EP 3 859 012 A1**
(43) Date of publication of application: **04.08.2021**
(21) Application number: 20155111.6
(22) Date of filing: 03.02.2020
(51) Int. Cl.: C12Q 1/6806, C12Q 1/6844

(54) **METHODS FOR AMPLIFICATION OF GENOMIC DNA AND PREPARATION OF SEQUENCING LIBRARIES**

(71) Applicant: Albert-Ludwigs-Universität Freiburg, 79098 Freiburg (DE)
(72) Inventor: RIBA, Julian, 79117 Freiburg (DE); NIEMÖLLER, Jean Christoph, 79104 Freiburg (DE)
(74) Representative: Hertin und Partner Rechts- und Patentanwälte PartG mbB

(57) **Abstract**

The invention relates to a method of amplifying DNA of more than one samples for generating a sequencing library, the method comprising a.) providing for each of the more than one samples an individual reaction vessel comprising the DNA in single stranded form, b.) adding first primers to the reaction vessel to produce a reaction mixture, wherein the first primers comprise in a 5' to 3' orientation a constant sequence and a variable sequence, wherein the constant sequence comprises in a 5' to 3' orientation a first part with a sequence that can be common for the more than one samples and a second part with a sequence that is different for each of the more than one samples, c.) adding a DNA polymerase to the reaction vessel, d.) subjecting the reaction mixture to a temperature at which the first primers anneal to the single stranded DNA via the variable sequence, e.) subjecting the reaction mixture to a temperature at which the DNA polymerase extends the 3'-ends of the annealed first primers to produce first-round amplicons, f.) subjecting the reaction mixture to a temperature to produce single stranded amplicons, g.) subjecting the reaction mixture to a temperature at which free first primers anneal to the single stranded DNA and to the first-round amplicons via the variable sequence, h.) subjecting the reaction mixture to a temperature at which the DNA polymerase extends the 3'-ends of the annealed first primers to produce first-round amplicons from first primers annealed to the provided DNA and second-round amplicons from first primers annealed to first-round amplicons, i.) subjecting the reaction mixture to a temperature to produce single stranded amplicons, repeating the steps (g)-(i) to produce second-round amplicons of the provided DNA.

## Description

The invention relates to a method of amplifying DNA of more than one samples for generating a sequencing library, the method comprising a.) providing for each of the more than one samples an individual reaction vessel comprising the DNA in single stranded form, b.) adding first primers to the reaction vessel to produce a reaction mixture, wherein the first primers comprise in a 5' to 3' orientation a constant sequence and a variable sequence, wherein the constant sequence comprises in a 5' to 3' orientation a first part with a sequence that can be common for the more than one samples and a second part with a sequence that is different for each of the more than one samples, c.) adding a DNA polymerase to the reaction vessel, d.) subjecting the reaction mixture to a temperature at which the first primers anneal to the single stranded DNA via the variable sequence, e.) subjecting the reaction mixture to a temperature at which the DNA polymerase extends the 3'-ends of the annealed first primers to produce first-round amplicons, f.) subjecting the reaction mixture to a temperature to produce single stranded amplicons, g.) subjecting the reaction mixture to a temperature at which free first primers anneal to the single stranded DNA and to the first-round amplicons via the variable sequence, h.) subjecting the reaction mixture to a temperature at which the DNA polymerase extends the 3'-ends of the annealed first primers to produce first-round amplicons from first primers annealed to the provided DNA and second-round amplicons from first primers annealed to first-round amplicons, i.) subjecting the reaction mixture to a temperature to produce single stranded amplicons, repeating the steps (g)-(i) to produce second-round amplicons of the provided DNA.

### BACKGROUND OF THE INVENTION

In the field of single cell analysis, both scientific publications and analysis products have grown exponentially over the last 10 years. Single cell genome analyses are for example often used in cancer research and have great potential for tumor diagnostics and future personalized medicine.

In order to analyze the genome of individual cells or other sample comprising only very little amount of genomic DNA, the genomic DNA must first be amplified (whole-genome-amplification, WGA for short). From the amplified genomic DNA, DNA libraries are then prepared for subsequent DNA sequencing using Next-Generation-Sequencing (NGS sequencing library preparation).

The term library preparation refers to a method for providing DNA amplicons or fragments thereof that can be used in a NGS reaction. For the most commonly used Illumina sequencing-by-synthesis technology, such DNA fragments must be between 200 and 1000 bp long and must be flanked by sequencing adapters at both ends. Only fragments/amplicons with a known sequence (adapter A) at one end (5'-end) and another known sequence (adapter B) at the other end (3-end) can be sequenced by this technology. Accordingly, since most known WGA technologies result in DNA amplicons that are much longer than 1000 bp, the process of library preparation mostly requires DNA fragmentation and subsequent ligation of adapter sequences.

Both the WGA and the NGS library preparation are complex and cost-intensive procedures (due to the expensive reagents). The cost aspect is also a limiting factor for the broad application of genomic analysis of individual single cells.

Several methods are known in the art for WGA. US6673541 B1 describes a WGA method that is based on fragmentation of the genomic templated DNA molecules by restriction enzymes and subsequent PCR amplification (AMPLI1). However, the applied protocol is highly complex and the NGS sequencing libraries must be prepared individually for each cell or sample.

US6977148B2 describes an amplification method called Multiple Displacement Amplification (MDA) that uses phi29 DNA polymerase for isothermal amplification. Due to the high accuracy of the phi29 polymerase, this method usually has relatively low error rates. However, nonlinear amplification leads to uneven amplification of individual regions of the genome, which is problematic in two respects: First, the amplified DNA is not suitable for the analysis of copy number variations. Furthermore, the unevenly amplified DNA requires a higher sequencing depth (i.e. additional costs) because otherwise the under-represented sections of the genome are not captured during sequencing. MDA results in DNA fragments with an average length of 10 kb (10,000 base pairs), which have to be fragmented before NGS sequencing library preparation can occur. Also, NGS sequencing library preparation has be performed individually for each cell or sample.

A method called *Multiple Annealing and Looping Based Amplification Cycles* (MALBAC) performs in a first step a "quasi linear" pre-amplification with degenerated primers with handles at the 3' end leading to the accumulation of loop and hairpin structures, which are protected against further amplification. At elevated temperatures, the hairpin structures can then be denatured and, after the addition of further primers, exponentially amplified by PCR. However, WGA by MALBAC results in DNA fragments with an average length of about 2 kb (2,000 base pairs). Accordingly, amplification products resulting from MALBAC have to be fragmented before library preparation, so that barcoding of amplification fragments can only occur during or after library preparation. Therefore, pooling of amplified DNA from different cells/samples can only occur after library preparation and library preparation has to occur individually for each sample/cell.

In summary, all of these established WGA methods require fragmentation of amplified DNA before individual library preparation for each sample/single cell.

For fragmentation, the amplification products resulting from known WGA methods can be subjected to sonication or enzymatic cleavage. Subsequently, the fragmented amplicons can be ligated to sequencing adapters. In methods of the state of the art, the ligated sequencing adapter often comprise an individual barcoding-sequence which enables identification of DNA fragments resulting from an individual cell. However, since barcoding of the fragments only occurs after fragmentation, library preparation has to be performed individually for each sample/each cell to be analyzed. Cell-specific barcoding-sequences cannot be introduced during the initial WGA step using WGA methods of the state of the art, since such amplification methods result in fragments of 2.000 to 10.000 bp and subsequent fragmentation would lead to removal of barcoding-sequences introduced during WGA.

As explained above, during NGS library preparation two different adapter sequences are ligated to the 5'- and 3'-ends of the amplicons of 200 to 1000 bp length. If these two adapters can be attached to the fragments to be sequenced via a simple ligation, three permutations of ligated products can occur: One with adapter A at both ends, one with adapter B at both ends, and one with adapter A at one end and adapter B at the other end (asymmetry). This last product is the only desired product and thus, after ligation, further purification steps are required. Although technologies have been developed in order to ensure generation of asymmetric ligation products, these are not compatible with all kinds of WGA methods, which is why alternative methods for library preparation generating asymmetric ligation products are required.

In light of the known methods for WGA and NGS sequencing library preparation, there remains a significant need in the art for the provision of methods of amplifying genomic DNA of more than one sample resulting in amplified DNA fragments that do not have to be further fragmented for library preparation and that comprise an sequence that identifies them as originating from a specific sample, such as a specific single cell. Furthermore, there is a need in the art for alternative or improved methods for asymmetric ligation of sequencing adapters to DNA fragments.

### SUMMARY OF THE INVENTION

In light of the prior art the technical problem underlying the present invention is to provide a method of amplifying genomic DNA of more than one sample resulting in amplified DNA fragments that do not have to be further fragmented for library preparation and that comprise an sequence that identifies them as originating from a specific sample, such as a specific single cell.

An additional problem is the provision of an alternative or improved method for asymmetric ligation of sequencing adapters to DNA fragments.

These problems are solved by the present invention, in particular by features of the independent claims and the aspects and embodiments of the invention disclosed herein.

### Embodiments relating to a method of amplifying genomic DNA

The invention therefore relates to a method of amplifying DNA of more than one samples for generating a sequencing library, the method comprising
a. providing for each of the more than one samples an individual reaction vessel comprising the DNA in single stranded form,
b. adding first primers to the reaction vessel to produce a reaction mixture,
   wherein the first primers comprise in a 5' to 3' orientation a constant sequence and a variable sequence,
   wherein the constant sequence comprises in a 5' to 3' orientation a first part with a sequence that can be common for the more than one samples and a second part with a sequence that is different for each of the more than one samples,
c. adding a DNA polymerase to the reaction vessel,
d. subjecting the reaction mixture to a temperature at which the first primers anneal (hybridize) to the single stranded DNA via the variable sequence,
e. subjecting the reaction mixture to a temperature at which the DNA polymerase extends the 3'-ends of the annealed first primers to produce first-round amplicons,
f. subjecting the reaction mixture to a temperature to produce single stranded amplicons,
g. subjecting the reaction mixture to a temperature at which free first primers anneal to the single stranded DNA and to the first-round amplicons via the variable sequence,
h. subjecting the reaction mixture to a temperature at which the DNA polymerase extends the 3'-ends of the annealed first primers to produce first-round amplicons from first primers annealed to the DNA and second-round amplicons from first primers annealed to first-round amplicons,
i. subjecting the reaction mixture to a temperature to produce single stranded amplicons,
j. repeating the steps **(g)-(i)** to produce second-round amplicons of the DNA.

The invention preferably relates to the amplification of very small amount of DNA provided in each of the more than one samples. In embodiments, the method relates to the amplification of genomic DNA, such as genomic DNA from more than one single cells. Therefore, in embodiments, the invention relates to a method of amplifying genomic DNA of more than one samples for generating a sequencing library, the method comprising
a. providing for each of the more than one samples an individual reaction vessel comprising the genomic DNA in single stranded form,
b. adding first primers to the reaction vessel to produce a reaction mixture,
   wherein the first primers comprise in a 5' to 3' orientation a constant sequence and a variable sequence,
   wherein the constant sequence comprises in a 5' to 3' orientation a first part with a sequence that can be common for the more than one samples and a second part with a sequence that is different for each of the more than one samples,
c. adding a DNA polymerase to the reaction vessel,
d. subjecting the reaction mixture to a temperature at which the first primers anneal (hybridize) via the variable sequence to the single stranded genomic DNA,
e. subjecting the reaction mixture to a temperature at which the DNA polymerase extends the 3'-ends of the annealed first primers to produce first-round amplicons,
f. subjecting the reaction mixture to a temperature to produce single stranded amplicons,
g. subjecting the reaction mixture to a temperature at which free first primers anneal via the variable sequence to the single stranded genomic DNA and to the first-round amplicons,
h. subjecting the reaction mixture to a temperature at which the DNA polymerase extends the 3'-ends of the annealed first primers to produce first-round amplicons from first primers annealed to genomic DNA and second-round amplicons from first primers annealed to first-round amplicons,
i. subjecting the reaction mixture to a temperature to produce single stranded amplicons,
j. repeating the steps **(g)-(i)** to produce second-round amplicons of the genomic DNA.

The invention is directed to a method of amplifying genomic DNA of more than one samples, wherein during the initial amplification step the amplified DNA of each sample is labeled or tagged with an individual sequence-label (or an individual sequence barcode or an individual barcoding-sequence). Provision of a barcoding-sequence to the amplicons during the initial amplification step enables pooling of amplicons generated from the initial amplification before a sequencing library is generated by providing the amplicons with adapter sequences for sequencing. The provision of the sequence-labels is not disclosed for amplification methods and in particular WGA methods of the state of the art.

Known methods of genomic DNA amplification do not use primers comprising sequence-labels during the initial amplification step, since the generated amplicons are too long to be used directly in a sequencing reaction. Instead, such initial amplicons have to be fragmented to be short enough to be used in an Illumina sequencing reaction and during the fragmentation a sequence-label that has been incorporated by means of the primers used for the initial amplification would be removed from the amplicons.

In contrast, surprisingly it is possible by means of the method of the invention to generate amplicons of genomic DNA that are short enough to avoid an additional fragmentation step. The amplification method of the invention generates sufficient number amplicons of genomic DNA that have a maximum length of about 1000 nucleotides in order to cover the whole genome. Accordingly, the amplification method of the invention enables subsequent library preparation without a fragmentation step resulting in a sequencing library that covers the whole genomic DNA provided in each of the samples of the method of the invention. Therefore, it is possible to pool reaction mixtures resulting from the amplification method of the invention or amplicons resulting from the amplification method of the invention already after the initial amplification reaction and before library preparation. This makes it possible to prepare libraries of genomic DNA of several samples in a single reaction well, since amplicons of genomic DNA of each sample have been provided with a sequence-label during the initial amplification and can therefore be differentiated. Subsequent library preparation can therefore be performed on pooled samples and is not required to be performed individually, as in the methods of the state of the art.

It is a striking advantage of the method of the invention that the amplification products comprise sufficient amplicons of less than about 1000 bp length that cover the complete sequence of genomic DNA initially provided for each sample.

In embodiments of the method for amplifying genomic DNA of the invention, the more than one samples are more than one single cells. Such single cells can be prokaryotic cells (such as bacterial or Archaeal cells) or eukaryotic cells (such as mammalian or human cells). In case of single cells analysis, the cells can be first isolated by isolating them into the reaction vessels of a microtiter plate. Several methods can be used for separation, such as sorting with a flow sorter or a FACS device, single-cell dispensing, limiting dilution, or micromanipulation. The cells may then be lysed in a suitable lysis buffer (cell lysis), whereby the cell membrane is broken open so that the DNA molecules can be accessed for subsequent amplification. The genomic DNA is now available as the starting material (DNA template) for the subsequent reaction. The necessary reagents and a primer with a cell/well-specific barcoding-sequence can now be added to each well. This is followed by pre-amplification. After pre-amplification, which is also referred to as the first amplification reaction of the invention, the liquid from all wells can be pooled into a reaction vessel. The subsequent second amplification reaction and creation of the sequencing library by provision of asymmetric adapter sequences to the amplicons of the amplification method of the invention can now be performed in a single reaction vessel without the need for fragmentation of the amplicons.

In embodiments of the amplification method, samples comprise small amounts of genomic DNA that require an amplification step prior to use in a sequencing reaction. Samples comprising a single cell only comprise one set of chromosomes representing genomic DNA. Accordingly, there is only one copy of double stranded genomic DNA present in each sample comprising a single cell.

In order to sequence the genomic DNA present in a single cell, the DNA has to be amplified to provide an amount of DNA that is sufficient for sequencing. Ideally, the amplification reaction does not create a bias towards certain sequences by more efficiently amplifying certain sequences, while less efficiently amplifying other sequences of the provided genomic DNA. Many methods of amplifying genomic DNA create a bias towards certain sequences, which are amplified more efficiently in the beginning. Since these efficiently generated amplicons can in certain amplification methods also serve as templates in the next amplification round, a bias for such sequences is generated. However, in the context of the present invention it is possible to avoid such a bias, since the method of the invention enables a "quasi-linear" amplification that amplifies the whole genomic DNA equally efficient without creating a bias towards certain sequences. This is an essential feature for methods of amplifying genomic DNA for sequencing purposes, if quantitative results from the sequencing analysis are desired.

In embodiments, the method of the invention may comprise a sample that represents a negative control that does not comprise any genomic DNA or any DNA at all. Furthermore, a positive control sample may be included, for example a sample that comprises a known, defined amount of DNA, such as a defined amount of genomic DNA.

In order to provide the DNA, preferably genomic DNA, of a sample in single stranded form, the sample can be subjected to a temperature that leads to melting of the double stranded genomic DNA of the sample. This temperature is usually a temperature above 90 °C, such as for example 91, 92, 93, 94, 95, 96, 97, 98 or 99 °C. In the context of the invention, it preferably about 92 °C to 98 °C.

In embodiments, for example such embodiments where the one or more samples of the method of amplifying genomic DNA are individual single cells, the step of providing single stranded genomic DNA may comprise cell lysis and/or chromatin digestion. The step of cell lysis can also be regarded as an individual method step that occurs in the beginning of the method. For cell lysis, a suitable volume of a lysis buffer may be added to each sample. The samples may be mixed and/or heated to an elevated temperature in the range of 60-100 °C. A suitable protease for digestion of the protein components of chromatin may be added to the sample followed by an incubation at a suitable temperature and for a suitable time in order to remove protein components from the DNA material of the samples.

In embodiments of the invention, the order of the certain method steps may be modified. Possible modifications of the method step order are obvious to a person skilled in the art. For example, method steps (a)-(c) may have a different order, since it is obviously possible to first assemble a reaction mixture comprising first primers (b) and a polymerase (c) and DNA, such as genomic DNA, and subsequently bring the reaction mixture to a temperature at which the provided DNA, which may be double stranded genomic DNA, is broad into a single stranded state (reaction step a). Accordingly, the addition of first primers and a polymerase can occur before heating the sample so that the comprise dsDNA is provided in a single stranded state.

The first primer may be added to the sample prior to subjecting the sample to a temperature leading to the melting of double stranded DNA. Similarly, the DNA polymerase may have been added to the sample before the melting step, preferably at the same time as the first primer. The DNA polymerase to be used in the context of the method of the invention is preferably stable at temperatures up to 100 °C, such as for example a Taq polymerase or a Deep Vent DNA Polymerase or a Deep Vent (exo-) DNA Polymerase, which is preferably used in the context of the method of the invention. Preferably, the DNA polymerase of the initial amplification step of the method of the invention is a Deep Vent (exo-) DNA Polymerase with high stability at 95°C and 100 °C and eliminated 3' to 5' proofreading exonuclease activity.

Polymerases to be used in the context of the invention comprise strand-displacing polymerases, polymerases that possess a 5'-flap endonuclease and/or polymerases 5'-3' exonuclease activity. Strand-displacing polymerases are polymerases that will dislocate downstream fragments as it extends. Strand displacing polymerases include Φ29 Polymerase, Bst Polymerase, Pyrophage 3173, Vent Polymerase, Deep Vent polymerase, TOPOTaq DNA polymerase, Vent (exo-) polymerase, Deep Vent (exo-) polymerase, 9°Nm Polymerase, Klenow fragment of DNA Polymerase I, MMLV Reverse Transcriptase, AMV reverse transcriptase, HIV reverse transcriptase, a mutant form of T7 phage DNA polymerase that lacks 3'-5' exonuclease activity, or a mixture thereof. One or more polymerases that possess a 5' flap endonuclease or 5 '-3' exonuclease activity such as Taq polymerase, Bst DNA polymerase (full length), E. coli DNA polymerase, LongAmp Taq polymerase, OneTaq DNA polymerase or a mixture thereof may be used to remove residual bias due to uneven priming.

In embodiments of the invention, the initial amplification reaction comprises a polymerase with 5'-3' exonuclease activity and/or a polymerase with strand displacement activity.

Deep Vent® (exo-) DNA Polymerase (NEB) exhibtis no 5'-3' exonuclease activity, strong strand displacement activity and high thermostability. SD Polymerase (Bioron GmbH) also exhibits strand displacement activity, no 5'-3' exonuclease activity and thermostability up to 93°C. Both are suitable polymerases for the method of the invention.

The **first primers** comprise in a 5' to 3' orientation a constant sequence and a variable sequence. The constant sequence is located upstream (towards the 5'-end) from variable sequence. In embodiments of the first primers, the constant sequence is located at the 5'-end of the first primers. In embodiments, the sequence of the first primers consists a variable sequence at the 3'-end and a constant sequence at the 5'-end.

In embodiments, the variable sequence of the first primers comprises between 3 and 12 nucleotides, which are randomly selected from among G, T, A and C, wherein the variable sequence is configured to enable the first primers to anneal randomly to single-stranded DNA molecules, preferably single-stranded genomic DNA molecules.

In further embodiments, the variable sequence of the first primers consists of between 3 and 12 nucleotides, which are randomly selected from among G, T, A and C, wherein the variable sequence is configured to enable the first primers to anneal randomly to single-stranded DNA molecules, preferably single-stranded genomic DNA molecules. In further embodiments, the variable sequence consists for 4-11, 5-10, 6-9, 7-8 nucleotides, wherein the indicated ranges include the noted end-values.

In further embodiments, the variable sequence of the first primers consists of between 3 and 12 nucleotides, whereof 1-6 nucleotides are LNA nucleotides. In embodiments, the variable sequence consists of LNA nucleotides. In embodiments, the LNA percentage in the variable sequence is 10, 20, 30, 40, 50, 60, 70, 80, 90 or 100 %. Such embodiments comprising LNA nucleotides are advantageous since LNA nucleotides have a stabilizing effect.

In further embodiments, the variable sequence of the first primers consists of between 3 and 12 nucleotides, where parts of the variable sequence contain bases randomly selected from among G and C and in other parts of the variable sequence are randomly selected from among G, T, A and C. In embodiments, the variable sequence of the first primers are GC-rich, which has a stabilizing effect and/or increases primer affinity. In embodiments, the GC-percentage in the variable sequence is above 60, 65, 70, 75, 80, 85, 90, 95 %.

In embodiments, the length of the variable sequence is in the range of 6-18, preferably 7-17, 8-16, 9-15, 10-14, 11-13 or 12 nucleotides. In such embodiments, certain or all nucleotides of the variable sequence may be LNA nucleotides. Such variable sequence may also be GC-rich.

Comparably long variable sequences, such as variable sequences of at least 6, 7, 8, 9, 10, 11, 12, 13, 14 or more nucleotides have a stabilizing effect on primer binding and priming of the amplification reaction. Similarly, LNA nucleotides as well as a high GC-content in the variable sequence also stabilize primer binding and priming.

Experimental data indicate that surprisingly stabilization of first primer binding, for example through a comparably long variable sequence that may comprise LNA nucleotides and/or may have a high GC-content leads to amplification of shorter fragments which are mostly or almost all below 1000 nucleotides length.

In embodiments, the Mg concentration in the reaction mixture is about 0.5 - 5.0 mM. In a specific embodiment, the Mg concentration in the reaction mixture is about 3.5 mM. In further embodiments, the Mg concentration is above 4 mM, preferably above 4.5 mM. In embodiments, the Mg concentration is about 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 21, 22, 23, 24, 25, 26, 27, 28, 29, 30 mM.

Mg concentrations of 4 mM of more can lead to an increasing stability of binding of the first primers to the DNA via the variable sequence, which leads to the preferential generation of nucleotides of 1000 nucleotides length or less.

In embodiments, the reaction mixture comprises betaine, preferably at a concentration of about 0,5 - 2 M. In a specific embodiment, the betaine concentration in the reaction mixture is about 1 M. In further embodiments, the betaine concentration is below 1 M, preferably below 0.9, 0.8, 0.7, 0,6, 0.5, 0.4, 0.3, 0.2, 0.1, 0.09, 0.08, 0.07, 0.06, 0.05 M.

Betaine concentrations of less than 1 M can lead to an increasing stability of binding of the first primers to the DNA via the variable sequence, which leads to the preferential generation of nucleotides of 1000 nucleotides length or less.

In embodiments, the first primers can initiate overlapping amplicons throughout the genome. In embodiments, the variable sequence may consist of a first part and a second part, wherein the second part is located at the 3'-end of the first primer.

In embodiments, the variable sequence may be randomly selected or may be purposefully selected commensurate with the frequency of its representation in a source DNA, such as genomic DNA. In specific embodiments, the nucleotides of the variable sequence will prime at target sites in a source DNA, such as a genomic DNA, containing the corresponding Watson-Crick base partners. In a particular embodiment, the variable region is considered degenerate.

The first part of the variable sequence may be randomly selected or may be purposefully selected commensurate with the frequency of its representation in a source DNA, such as genomic DNA. In specific embodiments, the nucleotides of the first part of the variable sequence will prime at target sites in a source DNA, such as a genomic DNA, containing the corresponding Watson-Crick base partners. In a particular embodiment, the first part of the variable region is considered degenerate.

The first part of the variable sequence can be characterized by a plurality of nucleotides, such as between 3 and 7 nucleotides which are randomly selected from among G, T, A, and C. For example, if the first part of the variable sequence includes 5 nucleotides, then the number of possible random sequences forming primers is 4⁵.

The second part of the variable sequence can include or can be composed of between 2 and 4 nucleotides, wherein the second part of the variable sequence avoids self-complementary sequences. For example, the second part of the variable sequence can include three G (i.e., G-G-G) or three T (i.e., T-T-T) or TTG, GAA or ATG.

The constant sequence of the first primers comprises in a 5' to 3' orientation a first part with a sequence that can be common for the more than one samples and a second part with a sequence that is different for each of the more than one samples.

In embodiments, the constant sequence of the first primers comprises between 10 and 70 nucleotides, preferably about 10-40 nucleotides, wherein the first part of the constant sequence comprises between 5 and 66 nucleotides, preferably 5-36 nucleotides and the second part of the constant sequence comprises between 4 and 10 nucleotides.

The constant sequence of the first primer may comprise 10-80, 11-79, 12-78, 13-77, 14-76, 15-75, 16-74, 17-73, 18-72, 19-71, 20-70, 21-69, 22-68, 23-67, 24-66, 25-65, 26-64, 27-63, 28-62, 29-61, 30-60, 59-31, 58-32, 33-57, 34-56, 35-55, 36-54, 37-53, 38-52, 39-51, 40-50, 41-49, 42-48, 43-47, 44-46, 45 nucleotides, wherein the indicated ranges include the noted end-values. This holds true for all end-values disclosed herein in the description of a parameter range.

Possible length of the first part and the second part of the constant sequence can be calculated accordingly. The second part of the constant sequence can be, for example, 4, 5, 6, 7, 8, 9, 10, 11 or 12 nucleotides long.

The second part of the constant sequence of the first primers represents the sequence-label (or barcoding-sequence) that is specific for each sample of the method of the present invention. By means of the second part of the constant sequence, amplicons can be assigned to individual samples also after pooling of amplicons after the initial round amplification step using the first primers.

In embodiments of the method for amplifying genomic DNA of the invention, the sequence of the first part of the constant sequence of the first primers is common for all of the more than one samples. Such embodiments are preferable, since the amplicons resulting from all samples of the method have the same 5' and 3' ends, which can be targeted by further primers or oligonucleotides used for amplification reactions or library preparation. This enables pooling of the reaction mixtures of the individual samples as early as after method step (j).

The constant sequence can include a nucleic acid sequence that is substantially non-self-complementary and substantially non-complementary to other primers in the plurality of primers used in the reaction mixture. The constant sequence is preferably known and may be a targeted sequence for a primer in amplification or library preparation methods.

According to one aspect, the first part of the constant sequence of the first primers is common to all first primers used in the context of the method of the invention.

In embodiments, the constant sequence or the first part of the constant sequence and is characterized by a plurality of nucleotides, which include G, T, and A, but excluding C. That is, C is not present in the constant sequence or the first part of the constant sequence. In embodiments, C is not present in the second part of the constant sequence.

In one embodiment of the invention, the first primers have a sequence according to SEQ ID NO1, wherein D can be selected from G, A and T, and N can be selected from G, A, T and C.

| **SEQ ID** | **Sequence** | **Description** |
|---|---|---|
| SEQ ID NO1: | ATGGAGAGTGATTGGAGAGGTAGTGCGAAGDDDDDNNNN NNN | First Primer #1 (Primer A) |

In embodiments, the first primers are HPLC purified oligonucleotides. Preferably, the primers are phosphorylated at the 5'-end. In the embodiment of SEQ ID NO1, the first part of the constant sequence of the first primer consists of 30 nucleotides, which can be selected among A, T, G and C. The second part of the constant sequence consists of 5 nucleotides. These can be selected among A, T, G and C. In preferred embodiments the nucleotides of the second part of the constant sequence can be G, A or T. The variable sequence at the 3'-end consists of 7 nucleotides which are randomly selected among A, G, T and C. In embodiments, the variable sequence of first primers of SEQ ID NO1 comprises or consists of LNA nucleotides. In embodiments of the invention, the primer according to SEQ ID NO1 comprises phosphrothioate modifications (phosphrothioate internucleotide linkages) between all nucleotides of the second part of the constant sequence.

In specific embodiments of the method for amplifying genomic DNA of the invention, the first primers comprise in the constant sequence, preferably at the 3'-end of the first part of the constant sequence and/or in the second part of the constant sequence, preferably at the 5'-end of the second part of the constant sequence modified nucleotides resistant to a 5' to 3' exonuclease, such as nucleotides with a phosphorothioate modification or LNA nucleotides. Preferably all nucleotides of the second part of the constant sequence of the first primers are modified.

Such embodiments are particularly useful in case the amplicons resulting from the method of amplifying genomic DNA of the present invention are used as starting material for the method of generating a sequencing library of the present invention. This method of preparing a sequencing library involves subjecting the DNA amplicons or fragments to be sequenced to a 5'-exonuclease treatment. By using primers comprising the nucleotide modifications described herein, it can be ensured that the exonuclease is not removing the barcoding-sequence incorporated into the amplicons.

The second part of the constant sequence of the first primers represent a sequence-label, which may also be called a barcoding-sequence, which is different for each kind of first primers that are used for different sample, such as different cells, in the context of the present invention. In contrast, the first part of the constant sequence is the same for each kind of first primers, which makes it possible to use the amplicons generated for each sample in the first amplification step of the method of the invention in a pooled fashion for subsequent processing steps that use the first part of the constant sequence.

In exemplary embodiments, the second part of the constant sequence of multiple kinds of primer A that can be used for different samples in parallel initial amplification reactions of the method of the invention consist or comprise one of the sequences, which comprise nucleotides selected from A, T and G, disclosed in *Table 1.*

**Table 1. Examples of barcoding-sequences (second part of the constant sequence) of first primers of the invention.**

| **SEQ ID NO** | **Sequence** | **SEQ ID NO** | **Sequence** | **SEQ ID NO** | **Sequence** |
|---|---|---|---|---|---|
| 11 | AAAGA | 19 | ATGAT | 27 | TAAGT |
| 12 | AAATG | 20 | ATTGA | 28 | TAGAT |
| 13 | AAGAA | 21 | ATTTG | 29 | TATGA |
| 14 | AATGT | 22 | GATTT | 30 | TATTG |
| 15 | AGATA | 23 | GTAAT | 31 | TGAAT |
| 16 | AGTAT | 24 | GTATA | 32 | TGTTA |
| 17 | ATAAG | 25 | GTTAA | 33 | TTAGA |
| 18 | ATAGT | 26 | TAAAG | 34 | TTATG |

After the first primers and preferably also the DNA polymerase have been added to the reaction mixtures of each sample of the method of amplifying genomic DNA of the invention, the reaction mixture is subjected to a temperature at which the first primers anneal (hybridize) via the variable sequence to the single stranded genomic DNA. To this end, the reaction mixture, which may have been at a temperature above 90 °C before in order to provide single stranded genomic DNA, is cooled down to a temperature suitable for annealing of the first primers to the single stranded DNA of the samples. In embodiments the reaction mixture is broad to a temperature at which annealing of the first primers to the single stranded DNA takes place, for example a temperature in the range of 0-30 °C, preferably a temperature in the range on 4-25 °C. In embodiments, the reaction mixture remains at the annealing temperature for about 20-120 seconds, preferably 25, 30, 35, 40, 45, 50, 55, 60, 65, 70, 75, 80, 85, 90 seconds.

Subsequently, the temperature may be elevated to a temperature at which the DNA polymerase extends the 3'-ends of the annealed first primers to produce first-round amplicons. The temperature at which this DNA amplification take place may be in the range of about 30-75 °C. The amplification period may be in the range of about 0.5 - 10 minutes (min), such as for example about 1, 1.5, 2, 2.5, 3, 3.5, 4, 4.5, 5, 5.5, 6, 6.5, 7, 7.5, 8, 8.5, 9, 9.5 or 10 minutes. During the amplification period, the temperature at which amplification takes place, which is the temperature at which the DNA polymerase extends the 3'-ends of the annealed first primers to produce first-round amplicons, may be subsequently elevated within the suitable temperature range. For example, the sample may initially be broad to 30 °C for 45 seconds, subsequently to 40 °C for 45 seconds, to 50 °C for 45 seconds and finally to 70 °C for 120 seconds.

In embodiments, the method steps of (d) and (e) and (g) and (h) may be achieved by subjecting the reaction mixture to a temperature gradient, such as a gradient of 10-70°C. In embodiments, the temperature increasing rate may be 0.1 °C/minute. In further embodiments, the temperature increasing rate may be 0.1 °C/second. Suitable increase rates can be determined by a skilled person for a particular application of the method of the invention.

In a specific embodiment, the reaction mixture may be first quickly cooled down, for example to about 0-10°C and subsequently be subjected to a temperature gradient of 10-70°C for primer annealing and amplification. Therein, the reaction mixture may remain at 10°C for about 2 minutes, is subsequently broad to 20°C with an increase rate of about 0.3°C/second, remains at 20°C for about 40-60, preferably 50 seconds, is subsequently broad to 30°C with an increase rate of about 0.3°C/second, remains at 30°C for about 40-60, preferably 45 seconds, is subsequently broad to 40°C with an increase rate of about 0.3°C/second, remains at 40°C for about 40-60, preferably 45 seconds, is subsequently broad to 50°C with an increase rate of about 0.3°C/second, remains at 50°C for about 40-60, preferably 45 seconds, and is subsequently broad to 70°C with an increase rate of about 3°C/second, and remains at 70°C for about 2 minutes. Alternative suitable temperature gradient programs can be developed by a skilled person, especially in view of the described specific embodiment. The applied temperature programs for annealing and amplification may be modified in view of a particular experimental or routine application of the invention, as can be determined by a skilled person.

The initial amplification/primer-extension step of the method of the invention leads to the production of first-round amplicons. These amplicons of the provided DNA material, preferably genomic DNA, are generated by extension of the free 3'-ends of the first primers which have (randomly) annealed to the (genomic) single-stranded DNA via the variable sequence. Since the first primers in the reaction mixture of each sample comprise a high number of different primers with different variable sequences, the primers can bind at multiple sites of the provided DNA material.

After extension of the free 3'-ends during the amplification step, the reaction mixtures are subjected to a temperature to produce single stranded amplicons. This is a temperature suitable for melting the extended first primers and the provided DNA material to which the primers annealed, and which severed as a template for primer extension/amplification. This temperature may be in the range of 85-100 °C, preferably 88-98, 90-96, 91-95, 92-94 or 93 °C. The reaction remains at this temperature for a period sufficient to produce single stranded DNA material, preferably single stranded genomic DNA. This period is preferably in the range of 20-60, 25-55, 30-50, 35-45 or about 30 seconds.

This melting step of the amplification method of the invention is followed by subjecting the reaction mixture to a temperature at which free first primers anneal via the variable sequence to the single stranded (genomic) DNA and to the first-round amplicons, preferably to the 3'-end of the first-round amplicons. In embodiments, the temperature and time of this step correspond to the parameters described above for the annealing of the first primers to the single stranded genomic DNA. Since after the initial primer extension/amplification step the reaction mixture also comprises first-round amplicons, which are extended first primers that have randomly annealed to the provided DNA, in this annealing step the available free first primers can anneal not only to single stranded (genomic) DNA initially provided in the sample, but also to the single stranded first-round amplicons.

After annealing of the first primers, the reaction mixture is again subjected to a temperature at which the DNA polymerase extends the 3'-ends of annealed first primers. During this second amplification step first-round amplicons are generated from extension of first primers that have annealed to genomic DNA provided in the initial sample and second-round amplicons are generated from first-primers that have annealed to first-round amplicons.

First-round amplicons comprise at their 5'-end the sequence of the first-round primer including the constant sequence of a first primer (including the barcoding-sequence and the first part of the constant sequence). Second-round amplicons are generated from first-round primers that have annealed to a first-round amplicon, for example near their 3'-end or in a central region of the first-round amplicons, by extension of the free 3'-end of the annealed first primer, resulting in a primer extension product that comprises at its 3'-end a sequence that is complementary to the sequence at the 5'-end of a first-round amplicon. Accordingly, a second-round amplicon comprises at its 5'-end a constant region of the first primer of the respective sample and at its 3'-end a sequence complementary to the constant region of the first primer of the respective sample. After the amplification step leading to the generation of first- and second-round amplicons, the reaction mixture is subjected to a temperature to produce single stranded amplicons, as described above.

The steps (g.) subjecting the reaction mixture to a temperature at which free first primers anneal via the variable sequence to the single stranded genomic DNA and to the 3'-end of the first-round amplicons, (h.) subjecting the reaction mixture to a temperature at which the DNA polymerase extends the 3'-ends of the annealed first primers to produce first-round amplicons from first primers annealed to genomic DNA and second-round amplicons from first primers annealed to first-round amplicons, and (i.) subjecting the reaction mixture to a temperature to produce single stranded amplicons, are repeated several times in order to generate amplicons of the (genomic) DNA provided in the sample that can be used for a subsequent sequencing reaction. Preferably, the number of repetitions of steps g-j is in the range of 3-20, 4-19, 4-18, 5-17, 5-16, 6-15, 7-14, 8-13, 9-12 or 10-11 cycles.

In preferred embodiments of the method of amplifying genomic DNA of the invention, between step (i) and (j) the reaction mixture is subjected to a temperature at which second-round amplicons form a hairpin structure through hybridization of the complementary constant sequences at the 5' and 3' end of the second-round amplicons.

As explained above, the second-round amplicons comprise at their 5'- and 3'-ends complementary sequences that enable formation of a hairpin or panhandle structure by the second-round amplicons. Therein, the 5'-end anneals to or hybridizes to the 3'-end of the same second-round amplicon.

By including a step of subjecting the reaction mixture to a temperature at which second-round amplicons form such a hairpin structure through hybridization of the complementary constant sequences at the 5' and 3' end of the second-round amplicons between the step (i) of subjecting the reaction mixture to a temperature to produce single stranded amplicons, and step (g) the subsequent subjection of the reaction mixture to a temperature at which free first primers anneal to the single stranded genomic DNA and to the first-round amplicons in the amplification cycle of the method of amplifying (genomic) DNA of the invention, the second-round amplicons can be withdrawn from the amplification reaction and do not serve as templates in the next amplification step. A temperature at which second-round amplicons form a hairpin structure can be any temperature in the range of 50-80 °C. However, further suitable temperatures can be identified by a skilled person without undue experimentation. This temperature in particular also depends on the length of the constant region of the first primers forming the complementary sequences. In general, it can be expected that the longer the complementary sequence the higher can be the temperature at which second-round amplicons form a hairpin structure.

The inclusion of this additional step of subjecting the reaction mixture to a temperature at which second-round amplicons form such a hairpin structure through hybridization of the complementary constant sequences at the 5' and 3' end of the second-round amplicons in the amplification cycle of the amplification method of the invention is particularly advantageous, since amplicons, which represent regions of the provided (genomic) DNA that have already been amplified are withdrawn from the reaction and cannot serve as a template in subsequent amplification cycles. This prevents the creation of a bias towards DNA sequences that are most efficiently amplified and results in a quasi-linear initial amplification of the provided (genomic) DNA.

In further embodiments, the method of amplifying genomic DNA of more than one samples of the invention, comprises additionally
k. adding to the reaction mixture of step (j) second primers comprising at their 3'-end a sequence comprised by the first part of the constant sequence of the first primers,
I. subjecting the reaction mixture to a temperature to produce single stranded amplicons,
m. subjecting the reaction mixture to a temperature at which the second primers anneal to the first part of the constant sequence of the second-round amplicons,
n. subjecting the reaction mixture to a temperature at which the DNA polymerase extends the 3'-ends of the annealed second primers to produce double stranded third-round amplicons,
o. subjecting the reaction mixture to a temperature to produce single stranded amplicons,
p. repeating the steps **(m)-(o)** to produce double stranded third-round amplicons.

It is preferably that after the initial, preferably quasi-linear amplification of the provided (genomic) DNA, second primers are added to the reaction mixture for a second amplification reaction as described by method steps (k)-(p).

Therein, the second primers comprise at their 3'-end a sequence that enables hybridization and/or annealing of a second primer to a sequence that is complementary to a sequence comprised by the first part of the constant sequence of the first primers. This may be achieved by using second primers that comprise at their 3'-end a sequence comprised by the first part of the constant sequence of the first primers.

In embodiments, the nucleotide sequence of the second primers can consist of a sequence that enables hybridization and/or annealing of a second primer to a sequence that is complementary to a sequence comprised by the first part of the constant sequence of the first primers. In embodiments, the nucleotide sequence of the second primers consists of a sequence comprised by the first part of the constant sequence of the first primers. In embodiments, the sequence of the second primers is identical to the sequence of the first part of the constant sequence of the first primers.

In one embodiment of the invention, the second primers have a sequence according to SEQ ID NO2. Such second primers can be used in conjunction with first primers that have a sequence according to SEQ ID NO1.

| **SEQ ID** | **Sequence** | **Description** |
|---|---|---|
| SEQ ID N02: | ATGGAGAGTGATTGGAGAGGTAGTGCGAAG | Second Primer #1 (Primer B) |

In embodiments, the second primers are HPLC purified oligonucleotides. Preferably, the primers are phosphorylated at the 5'-end.

In embodiments, the second primers have a sequence length of about 10-70 nucleotides, such as 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 nucleotides. It is preferable that second primers have a sequence length in the range of 20-40, more preferably 25-35 nucleotides.

In embodiments, the second primers consist of a sequence comprised by the first part of the constant sequence of the first primers, or that enables hybridization to a sequence that is complementary to a sequence comprised by the first part of the constant sequence of the first primers, wherein the second primers preferably consist of 10-66 nucleotides, more preferably of 20-40 nucleotides, most preferably of about 25-35 nucleotides.

In embodiments of the invention, the second primers, such as a primer having a sequence according to SEQ ID NO2, comprise near their 3'-end, preferably within the first 10 nucleotides from their 3'-end, preferably at their 3'-end, at least one nucleotide that is resistant to a 5'-exonuclease, such as one or more a nucleotides with a phosphorothioate internucleotide linkages between.

In specific embodiments of the method for amplifying genomic DNA of the invention, the second primers comprise in the sequence at their 3'-end, preferably at their 3'-end, modified nucleotides resistant to a 5'-exonuclease, such as nucleotides with a phosphorothioate modification or LNA nucleotides, wherein preferably 1-6, 2-5 or 3-4 nucleotides comprised by the sequence at the 3'-end of the second primers are modified, more preferably the 1-6, 2-5 or 3-4 nucleotides at the 3'-end of the second primers are modified.

Such embodiments are particularly useful in case the amplicons resulting from the method of amplifying genomic DNA of the present invention are used as starting material for the method of generating a sequencing library of the present invention. This method of preparing a sequencing library involves subjecting the DNA amplicons or fragments to be sequenced to a 5'-exonuclease treatment. By using primers comprising the nucleotide modifications described herein, it can be ensured that the exonuclease is not removing the barcoding-sequence incorporated into the amplicons.

In further embodiments of the method of amplifying genomic DNA of the invention, the first and second primers are phosphorylated at their 5'-ends. This ensures that the amplicons of the method of amplifying genomic DNA of the invention are accessible to a 5'-exonuclease, which is required for performing the method of generating a sequencing library of the present invention described below.

In embodiments, the reaction mixture of step (j) that underwent multiple cycles of initial (preferably quasi-linear) amplification is subjected to a second amplification reaction. To this end, the reaction mixture of step (j) is subjected to a temperature to produce single stranded amplicons. This temperature is to be understood as a temperature at which not only hairpin structures formed by second-round amplicons, but also double stranded amplicons or hybrids of amplicons and genomic DNA are melted into single stranded molecules. The reaction mixture should stay at this melting temperature for a time sufficient to ensure complete melting of all hybridized DNA molecules of the reaction mixture, for example a time in the range of 20-120 seconds, such as 25, 30, 35, 40, 45, 50, 55, 60, 65, 70, 75, 80, 85, 90, 95, 100, 105, 110 or 115 seconds.

Prior or after this melting step, second primers are added to the reaction mixture.

The initial melting step is followed by subjecting the reaction mixture to a temperature at which the second primers anneal to the first part of the constant sequence of the second-round amplicons. Since the second primers can be understood as representing standard PCR primers that are complementary to the 3'-ends of the second-round amplicons of the reaction mixture, a skilled person is able to identify a suitable annealing temperature. For example, in case of second primers of about 30 nucleotide length, a suitable annealing temperature can be in the range of 50-70 °C, such as 51, 52, 53, 54, 55, 56, 57, 58, 59, 60, 61, 62, 63, 64, 65, 66, 67, 68 or 69 °C. Suitable times of the annealing step can be defined by a skilled person on the specific conditions, and include, without limitation, times in the range of 10-60 seconds, such as 10, 15, 20, 25, 30, 35, 40, 45, 50, 55 or 60 seconds.

After annealing of the second primers to the 3'-ends of the second-round amplicons, the reaction mixture is subjected to a temperature at which the DNA polymerase of the reaction mixture extends the 3'-ends of the annealed second primers to produce double stranded third-round amplicons. For this amplification reaction, the one or more polymerases added before the initial amplification step may be used, or additional polymerase may have been added before the second amplification reaction. The temperature depends on the employed polymerase and its ideal or suitable working temperature. Many suitable polymerases operate fine at 72 °C, which may therefore represent a suitable temperature for extending free 3'-ends of annealed second primers. The time for the primer extension reaction/amplification reaction can be defined by a person skilled in the art and depends on the specific reagents, such as the polymerase used in the reaction. However, suitable times can be in the range of 0.75-10 minutes, 1-9, 1.5-8.5, 2-8, 2.5-7.5, 3-7, 3.5-6.5, 4-6, 4.5-5.5 or 5 minutes.

Subsequent to extension of annealed second primers, the reaction mixture is subjected to a temperature to produce single stranded amplicons (step (o)). This temperature may correspond to the initial melting temperature of the second amplification reaction leading to melting of all double stranded DNA structures comprised by the reaction mixture.

In preferred embodiments of the method of the invention for amplifying genomic DNA the temperature to produce single stranded amplicons of step (o) is a temperature at which hairpin structures formed by second-round amplicons dehybridize and form single-stranded amplicons, while double stranded third-round amplicons do not dehybridize. The melting temperature of a hairpin structures formed by second-round amplicons is lower than the melting temperature of fully hybridized third-round amplicons and can be determined by a person skilled in the art. This temperature is preferably below 90 °C, such as a temperature in the range of 78-89 °C. In embodiments, in step (o) a temperature of about 85 °C may be employed, at which fully hybridized double stranded DNA of more than about 200 nucleotides does not dehybridize.

Subsequently, the steps (m)-(o) of the second amplification reaction of the amplification method of the invention are repeated several times. This enables conversion of all single-stranded second-round amplicons, which may be present as a hairpin structure, to be converted to double-stranded third-round amplicons, which can be used for library preparation. The steps (m)-(o) can be repeated several times. A skilled person is aware or can determine of a suitable number of cycles. In embodiments, 3-15 cycles are performed, in preferred embodiments 5-8 cycles are performed.

In preferred embodiments, the amplicons generated by the method of the invention have an average length off less than 1000 nucleotides.

In embodiments, the amplicons generated by the method of the invention have a median length off less than 1000 nucleotides.

In embodiments, the method of the invention generates amplicons off less than 1000 nucleotides that cover the whole genomic DNA provided in the reaction vessel. Accordingly, the amplicons of less than 1000 nucleotides length of the method are sufficient for use in a NGS reaction after library preparation.

It is a great advantage of the method of amplifying genomic DNA of the present invention that a sufficient amount of double stranded third-round amplicons are generated that have an average length of about 1000 base pairs or less and that cover the whole sequence of the DNA initially provided in the sample. This enables quantitative sequence analysis of the small amounts of (genomic) DNA provided in each sample without an additional fragmentation step.

This surprising effect enables the direct use of the generated amplicons for library preparation without having to further fragment the generated amplicons. This enables incorporation of barcoding-sequences into the amplicons already during the first amplification reaction of the method of the invention and therefore earlier than in any known method of amplifying genomic DNA of more than one single cells. Due to this early incorporation of sample/cell identifying barcodes into the generated amplicons, it is possible to pool the amplicons of several initial first amplification reactions at an early process step, such as after step (j) of the initial amplification reaction.

In embodiments of the method, after step (j) or (p) the reaction mixtures of the more than one samples are pooled in a single reaction vessel. In such embodiments, it is preferred that the sequence of the first part of the constant sequence is common for all of the more than one samples.

Pooling of the reaction mixtures of the more than one samples before library preparation is a great advantage of the amplification method of the present invention. Due to this feature it is possible to save a lot of time and reagents and therefore also costs when analyzing the genomic DNA sequences provided in several samples comprising small amounts of DNA, such as several single cells. It is possible to perform the library preparation for all cells in a single reaction. This is particularly advantageous for many applications that require the analysis of many single cells, preferably hundreds or even thousands of single cells, since thousands of handling steps can be reduced through labeling of the amplicons to be analyzed during the first amplification reaction and subsequent pooling of samples.

In embodiments, the amplification method of the invention involves an additional step of purifying pooled double-stranded third-round amplicons. To this end a magnetic bead purification method may be used, as described in the examples below.

### Embodiments of the invention relating to a method of generating a sequencing library

The present invention further relates to a method of generating a sequencing library comprising the steps of
a. providing in a reaction vessel double-stranded DNAs, such as dsDNA fragments or dsDNA amplicons,
b. generating from said double-stranded DNA double-stranded amplicons with overhanging single-stranded 3'-ends,
c. adding to the reaction vessel
   - gap-filling primers, wherein the gap-filling primers comprise
      o at their 3'-end a sequence that enables hybridization/annealing of the gap-filling primers to the overhang-sequences at the 3'-ends of the double stranded DNAs, and
      o a first adapter sequence for a subsequent sequencing of the double-stranded DNAs,
   - a DNA polymerase, and
   - a ligase,
d. subjecting the reaction mixture to a temperature at which
   - a gap-filling primer hybridizes to the overhanging single-stranded 3'-end of a dsDNA,
   - the 3'-end of the third primer is extended by the DNA polymerase and
   - the extended 3'-end of the gap-filling primer is ligated to a 5'-end of a strand of the dsDNA by the ligase.

The present invention further relates to a method of generating a sequencing library comprising the steps of
a. providing in a reaction vessel double-stranded DNAs, such as dsDNA fragments or dsDNA amplicons, wherein each strand of the dsDNAs comprises near its 5'-end, preferably within nucleotides 20-100, more preferably within the nucleotides 25-40 counted from the 5'-end, at least one modified nucleotide resistant to a 5'-exonuclease, such as nucleotides with a phosphorothioate modification or LNA nucleotides,
b. adding a 5'-exonuclease to the reaction vessel to produce a reaction mixture,
c. subjecting the reaction mixture to a temperature at which the 5'-exonuclease is active to generate dsDNA with overhanging single-stranded 3'-ends,
d. adding to the reaction vessel
   - gap-filling primers, wherein the gap-filling primers comprise
      o at their 3'-end a sequence that enables hybridization/annealing of the gap-filling primers to the overhang-sequences at the 3'-ends of the dsDNA, and
      o a first adapter sequence for a subsequent sequencing of the dsDNAs,
   - a DNA polymerase, and
   - a ligase,
e. subjecting the reaction mixture to a temperature at which
   - a gap-filling primer hybridizes to the overhanging single-stranded 3'-end of a double-stranded third-round amplicon,
   - the 3'-end of the third primer is extended by the DNA polymerase and
   - the extended 3'-end of the third primer is ligated to a 5'-end of a strand of the dsDNA by the ligase.

In the context of the invention, the method of generating a sequencing library can also be referred to as a method for asymmetric ligation of sequencing adapters to DNA fragments or amplicons. The present method of generating a sequencing library can use as a starting material any kind of double stranded DNA (dsDNA), such as amplicons resulting from a DNA amplification reaction, for example a PCR, which should be analyzed in a subsequent sequencing reaction. Such dsDNA can be genomic DNA, which has been fragmented. Furthermore, the dsDNA may originate from an amplification reaction employed to increase a small amount of DNA, such as a small amount of genomic DNA, which may have been provided by a single cell. The dsDNA provided for the method of generating a sequencing library may originate from an amplification reaction and may have undergone a fragmentation step before use in the method of the invention. The dsDNA may originate directly from an amplification reaction. In embodiments, the method relates to generating a sequencing a library, comprising providing double-stranded amplicons of genomic DNA as a starting material.

In embodiment, the average length of the provided dsDNA is less than 1000 nucleotides. In embodiment, the median length of the provided dsDNA is less than 1000 nucleotides. In embodiment, the provided dsDNA molecules are amplicons generated from an amplification of genomic DNA, wherein the amplicons comprise a number of amplicons of about 1000 nucleotides length or less that cover the whole genomic DNA that was provided as a starting material in the amplification reaction. Accordingly, the amplicons of about 1000 nucleotides length or less than 1000 nucleotides length of the method are sufficient for use in a NGS reaction after library preparation.

Each strand of the dsDNAs comprises near its 5'-end, preferably within nucleotides 20-100, more preferably within the nucleotides 25-40 counted from the 5'-end, at least one modified nucleotide resistant to a 5'-exonuclease (resistant nucleotide), such as nucleotides with a phosphorothioate modification or LNA.

5'-exonucleases are enzymes that work by cleaving nucleotides one at a time from the 5'-end (exo) of a polynucleotide chain by promoting a hydrolyzing reaction that breaks phosphodiester bonds at the 5' end. Addition of such an enzyme to a reaction vessel leads to stepwise removal of nucleotides from the 5' ends of both strands of a provided dsDNA. For some 5' to 3'-exonucleases it is required that the 5'-ends of the polynucleotide is phosphorylated. Therefore, it is preferred that the dsDNA provided in the method of generating a sequencing library of the invention is phosphorylated at the 5'-end.

In embodiments of the method of generating a sequencing library of the invention, the 5'-exonuclease is a lambda exonuclease and the 5'-ends of the provided double-stranded amplicons are phosphorylated at their 5'-ends.

Since each strand of the dsDNAs comprises near its 5'-end at least one nucleotide resistant to a 5'-exonuclease, addition of the 5'-exonuclease to the reaction mixture and subjection to a temperature at which the 5'-exonuclease is active leads to the generation of dsDNAs with free, overhanging single-stranded 3'-ends, since the complementary 5'-ends have been remove by the 5'-exonuclease until to the position where the one or more nucleotides resistant to the 5'-exonuclease are located.

The term "near the 5'-end" of a strand of dsDNA can relate to a length of about 20% of the total length of the strand, counted from the 5'-end. For example, for a strand of 1000 nucleotides, 20 % would correspond to the first 200 nucleotides counted from the 5'-end. In embodiments, the resistant nucleotide(s) are located within the first 100 nucleotides. In further embodiments, the one or more resistant nucleotides are located within about 90, 80, 70, 60, 50, 45, 40, 35, 30, 25, 20, 18, 16, 14, 12, 10, 9, 8, 7, 6, 5, 4, 3, 2 nucleotides from the 5'-end. Furthermore, in embodiments, the resistant nucleotide(s) is not located within the first 2, 3, 4, 5, 6, 7, 8, 9, 10, 11, 12, 13, 14, 15, 15, 17, 18, 19, 20, 21, 22, 23, 24, 25, 26, 27, 28 or 30 nucleotides from the 5'-end of the DNA strand. In embodiments, the resistant nucleotide(s) is within or between nucleotides 3--100, 4-99, 5-98, 6-97, 7-96, 8-95, 9-96, 10-95, 12-92, 14-92, 15-91, 16-90, 17-80, 18-70, 19-60, 20-50, 21-45, 22-42, 23-40, 24-38, 25-36, 26-35, 27-34, 28-33, 29-32 or 30-31, counted from the 5'-end.

It is preferably that there are more than one, preferably 2, 3 or 4 resistant nucleotides near the 5'-end of the DNA strands.

The temperature at which the 5'-exonuclease is active depends on the specific exonuclease and can be determined by a person skilled in the art. For example, a lambda exonuclease is active at 37 °C.

The time required for the exonuclease to generate the free 3'-ends is dependent on the specific exonuclease, the reaction temperature and the location of the resistant nucleotides. Preferably times are in the range of 1 minute to 8 hours, preferably 2-60 minutes, for example 30 minutes.

In embodiments, the 5'-exonuclease is inactivated by heating the reaction mixture to a suitable temperature, for example 75 °C or higher, such as over 90°C, after the free 3'-ends have been generated.

After generating free/overhanging single-stranded 3'-ends, gap-filling primers are added to the reaction vessel.

Gap-filling primers of the invention are designed to hybridize/anneal to the overhanging single-stranded 3'-ends of the provided dsDNAs. Preferably, the sequences at the ends of the provided dsDNAs are known and it is therefore possible to provide gap-filling primers that comprise at their 3'-end a sequence that enables annealing to the overhanging 3'-ends of the dsDNA, for example a sequence that is at least partially, preferably completely complementary to a sequence comprised by the overhanging 3'-end of the dsDNA.

Furthermore, the gap-filling primers comprise a first adapter sequence for subsequent sequencing of the dsDNAs of the sequencing library. The adapter sequence is preferably located at the 5'-end of the primer.

In embodiments, the sequence 3'-end a sequence that enables hybridization/annealing of the gap-filling primers to the overhang-sequences at the 3'-ends of the dsDNAs consists of 2-36 nucleotides, such as 2, 3, 4, 5, 6, 7, 8, 9, 10, 11, 12, 13, 14, 15, 16, 17, 18 ,19, 20, 21, 22, 23, 24, 25, 26, 27, 28, 29, 30, 31, 32, 33, 34 or 35 nucleotides. Furthermore, the adapter sequence, which is preferably located at the 5'-end of the gap-filling primers, can consist of 8-36, such as 9, 10, 11, 12, 13, 14, 15, 16, 17, 18 ,19, 20, 21, 22, 23, 24, 25, 26, 27, 28, 29, 30, 31, 32, 33, 34 or 35 nucleotides.

In embodiments, the sequence of the gap-filling primers consists of 20-100 nucleotides, such as 21, 22, 23, 24, 25, 26, 27, 28 ,29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 67, 68, 69, 70, 71, 72, 73, 74, 75, 76, 77, 78, 79, 80, 81, 82, 83, 84, 85, 86, 87, 88, 89, 90, 91,92, 93, 94, 95, 96, 97, 98 or 99 nucleotides.

In embodiments, the gap-filling primers have a sequence according to SEQ ID NO3 or SEQ ID NO6.

| **SEQ ID** | **Sequence** | **Description** |
|---|---|---|
| SEQ ID NO3 | AATGATACGGCGACCACCGAGATCTACACTCTTTCCCTAC AGAGAGGTAGTGC | Gap-filling Primer #1 (Primer C) |
| SEQ ID NO4 | GAGAGGTAGTGC | Sequence at the 3'-end of the gap-filling primer |
| SEQ ID NO5 | AATGATACGGCGACCACCGA | Illumina P5 Adapter sequence |
| SEQ ID NO6 | AATGATACGGCGACCACCGATAGCTCACATCTTTCCCTAC AGAGAGGTAGTGC | Gap-filling Primer #2 |

In embodiments, gap-filling primers are HPLC or PAGE purified oligonucleotides.

Furthermore, in the context of the method of generating a sequencing library of the invention, a DNA polymerase and a ligase are added to the reaction vessel comprising the reaction mixture. The DNA polymerase and the ligase can be added at the same time as the gap-filling primer after the exonuclease treatment. The DNA polymerase preferably does not have strand-displacement activity.

Upon subjecting the reaction mixture comprising dsDNAs with overhanging 3'-ends, the gap-filling primers, the DNA polymerase and the ligase to a suitable temperature or to a suitable temperature program (meaning a sequence of different temperatures), the gap-filling primers anneals to the overhang-sequences at the 3'-end of the dsDNAs, the DNA polymerase extends the 3'-ends of the gap-filling primers, and the ligase ligates the extended 3'-ends of the gap-filling primers to the 5'-ends of the dsDNAs. This process occurs at both ends of the dsDNAs on the opposing strands, so that the extended gap-filling primers are ligated to the 5'-end of each strand of the provided dsDNAs.

A temperature at which a gap-filling primer hybridizes to the overhanging single-stranded 3'-end of a double-stranded third-round amplicon, the 3'-end of the third primer is extended by the DNA polymerase without SD activity and the extended 3'-end of the third primer is ligated to the 5'-end of the double-stranded third-round amplicon by the ligase may relate to a suitable sequence of temperatures.

For example, the reaction mixture may be subjected to a temperature at which the gap-filling primer does not anneal to the free 3'-end, such as a temperature of about 70 °C or higher. This temperature depends on the length of the sequence at the 3'-end of the gap-filling primer and other parameters known to a skilled person and can therefore be adjusted as required. In embodiments, this relatively high temperature can be decreased continuously, for example at a rate of 0.1 °C/sec, until a temperature that ensures good activity of the DNA polymerase and the ligase. During the continuous decrease of the temperature, the gap-filling primers can anneal to the free 3'-ends of the dsDNA. Many DNA polymerases and ligases are highly active at 37 °C. so the Temperature may be decreased to 37 °C. Subsequently, the reaction mixture can remain at this temperature suited for DNA polymerase and ligase activity to ensure extension annealed gap-filling primers and subsequent ligation to the 5'-ends.

In preferred embodiments of method of generating a sequencing library of the invention the provided dsDNAs are amplicons of genomic DNA. Preferably, these amplicons have been generated from more than one samples, such as more than one single cells. In embodiments, the amplicons of each sample or cell comprise an individual sequence-label (barcoding-sequence), preferably of a length of 4-10 nucleotides. In that case, it is not required that barcoding-sequences are introduced to the amplicons during the present method of generating a sequencing library.

The sequence-label of the provided amplicons may be located near the 3'- and 5'-ends of the amplicons, preferably within nucleotides 20-110, more preferably within the nucleotides 25-50 counted from the 3'- and 5'-end. In embodiments, the sequence-label is located within the first 110 nucleotides. In further embodiments, the sequence-label is located within about 90, 80, 70, 60, 50, 45, 40, 35, 30, 25 or 20 nucleotides from the end. Furthermore, in embodiments, the sequence-label is not located within the first 8, 9, 10, 11, 12, 13, 14, 15, 15, 17, 18, 19, 20, 21, 22, 23, 24, 25, 26, 27, 28 or 30 nucleotides. In embodiments, the sequence-label is within or between nucleotides 15-100, 16-90, 17-80, 18-70, 19-60, 20-50, 21-45, 22-42, 23-40, 24-38, 25-36, 26-35, 27-34 or 28-33. A sequence label comprised by an amplicons of the method of generating a sequencing library is defined as the second part of a first primer, which is a barcoding-sequence, as disclosed herein.

In embodiments, the method of generating a sequencing library of the invention comprises after step (e)
f. adding adapter primers to the reaction vessel, wherein the adapter primers comprise
   - at their 3'-end a sequence that enables hybridization/annealing of the adapter primers to the 3'-ends of the strands of the provided dsDNAs, preferably with a higher affinity than the sequence at the 3'-end of the gap-filling primers, and
   - a second adapter sequence for a subsequent sequencing of the provided dsDNA,
g. subjecting the reaction mixture to a temperature to produce single stranded DNA (ssDNA),
h. subjecting the reaction mixture to a temperature at which the adapter primers anneal to the 3'-ends of the single-stranded provided DNA comprising ligated gap-filling primers at their 5'-end,
i. subjecting the reaction mixture to a temperature at which the DNA polymerase extends the 3'-ends of the annealed adapter primers and of the single-stranded DNA comprising ligated gap-filling primers to produce double-stranded adapter amplicons.

In the embodiment described above, adapter primers are added to the reaction mixture of step (e) of the method of generating a sequencing library. The adapter primers of the invention are designed to hybridize/anneal to the 3'-ends of the strands of the provided dsDNAs. Preferably, the sequences at the ends of the provided dsDNAs are known and it is therefore possible to provide adapter primers that comprise at their 3'-end a sequence that enables annealing to a 3'-ends of the provided dsDNAs, for example a sequence that is at least partially, preferably completely complementary to a sequence comprised by a 3'-end of the strands of the provided dsDNA. Preferably, the affinity of the adapter primers to a 3'-end of a strand of the provided dsDNA is higher than the affinity of the gap-filling primers to the 3'-end. For example, the sequence at the 3'-end of the adapter primers that is at least partially, preferably completely complementary to a sequence comprised by a 3'-end of the strands of the provided dsDNA comprises and is longer than the sequence at the 3'-end of the gap-filling primer.

Furthermore, the adapter primers comprise a second adapter sequence for subsequent sequencing of the dsDNAs of the sequencing library. The adapter sequence is preferably located at the 5'-end of the primer.

The method of generating a sequencing library of the invention is preferably for generating sequencing library to be used in a Illumina sequencing-by-synthesis sequencing method. Therefore, in embodiments, the first and second adapter sequences comprised by the gap-filling and adapter-primers of the method of the invention can be any adapter sequence known in the art and suitable for employing the dsDNA molecules resulting from the present method in such a reaction. For example, the first and second adapter sequences can be the P5 and P7 Illumina adapter sequence.

In embodiments, the adapter primers have a sequence according to SEQ ID NO7 or SEQ ID NO10, wherein N can be selected from G, A, T and C.

| **SEQ ID** | **Sequence** | **Description** |
|---|---|---|
| SEQ ID NO7 | CAAGCAGAAGACGGCATACGAGATNNNNNNCGAATGGAG AGTGATTGGAGAGGTAGTGCGAAG | Adapter Primer #1 (Primer D) |
| SEQ ID NO8 | ATGGAGAGTGATTGGAGAGGTAGTGCGAAG | Sequence at the 3'-end of the adapter primer |
| SEQ ID NO9 | CAAGCAGAAGACGGCATACGAGAT | Illumina P7 Adapter sequence (without index) |
| SEQ ID NO10 | CAAGCAGAAGACGGCATACGAGATNNNNNNCCTATGGAG AGTGATTGGAGAGGTAGTGCGAAG | Adapter Primer #2 |

In embodiments, adapter primers are HPLC or PAGE purified oligonucleotides. In embodiments, the gap-filling primers and/or the adapter primers do not contain a barcoding-sequence. In further embodiments, the gap-filling primers and/or the adapter primers do contain a barcoding-sequence. Inclusion of multiple barcoding sequences via one or more primers of the present invention can be advantageous for sequencing an even higher number of samples, such as single cells, in a single sequencing run. Multiple barcodes would enable unique dual indexing.

After the adapter primers have been added to the reaction mixture of method step (e), the reaction mixture is subjected to a temperature to produce ssDNA, preferably single-stranded amplicons. In order to ensure complete melting of the provided dsDNA, it is preferred to subject the reaction mixture to a temperature of more than 90 °C, preferably 91, 92, 93, 94, 95, 96, 97 or 98 °C. The initial incubation time at the melting temperature can be determined by a skilled person, for example at least 5 seconds, preferably at least 6, 7, 8, 9, 10, 11, 12, 13, 14, 15, 20, 25, 30, 35, 40, 45, 50, 55 or 60 seconds.

After melting of the dsDNA, the reaction mixture is subjected to a temperature at which the adapter primers anneal to the 3'-ends of the provide single stranded DNA comprising ligated gap-filling primers at their 5'-ends. Preferably, the adapter primers have a higher affinity to the 3'-ends of the provided single stranded DNA and therefore bind/anneal preferentially in comparison to access gap-filling primers that can still be present in the reaction mixture.

After the annealing step, which can be for example at a temperature in the range of about 60-70 °C and which lasts for example for 15-120 seconds, preferably about 40-80 seconds, the reaction mixture is subjected to a temperature at which a polymerase comprised by the reaction mixture extends the free 3'-ends of the annealed adapter primers as well as the free 3'-ends of the single-stranded provided DNA, for example a temperature of about 72 °C. The polymerase may be already comprised in the reaction mixture or may be added at the same time as or after adding the adapter primers.

Through extension of the adapter primers and the single-stranded DNA comprising the ligated gap-filling primer at its 5'-end, a dsDNA is generated wherein one strand comprises at its 5'-end the sequence of the adapter primer and at its 3'-end a sequence complementary to the gap-filling primer and the other strand comprises at its 5'-end the sequence of the gap-filling primers and at its 3'-end a sequence complementary to the adapter primer. Such dsDNAs are referred to as double-stranded adapter amplicons.

In embodiments the method of generating a sequencing library of the invention comprises after step (i)
j. subjecting the reaction mixture to a temperature to produce single stranded adapter amplicons,
k. subjecting the reaction mixture to a temperature at which the adapter primers and gap-filling primers anneal to the 3'-ends of the corresponding single-stranded adapter amplicons,
I. subjecting the reaction mixture to a temperature at which the DNA polymerase extends the 3'-ends of the annealed adapter and gap-filling primers to produce double stranded adapter amplicons
m. repeating the steps (j) - (I) to produce double stranded adapter amplicons.

In this embodiment, the reaction mixture of step (i) is subjected to a PCR protocol as known to a person skilled in the art, wherein the adapter primers and the gap-filling primers are serving as amplification primers. The reaction mixture comprising double-stranded adapter amplicons is subjected to a melting temperature for generating single-stranded adapter amplicons. Preferably, this temperature to produce single stranded adapter amplicons is a temperature of more than 90 °C, preferably 91, 92, 93, 94, 95, 96, 97 or 98 °C. The incubation time at the melting temperature can be determined by a skilled person, for example at least 5 seconds, preferably at least 6, 7, 8, 9, 10, 11, 12, 13, 14, 15, 20, 25, 30, 35, 40, 45, 50, 55 or 60 seconds.

After the melting step, the reaction mixture is subjected to a temperature at which adapter primers and gap-filling primers anneal to the 3'-ends of the corresponding single-stranded adapter amplicons. After the annealing step, which can be for example at a temperature in the range of about 60-70 °C and which lasts for example for 15-120 seconds, preferably about 40-80 seconds, the reaction mixture is subjected to a temperature at which a polymerase comprised by the reaction mixture extends the free 3'-ends of the annealed adapter primers as well as the free 3'-ends of the single-stranded provided DNA, for example a temperature of about 72 °C. The polymerase may be already comprised in the reaction mixture or may be added at the same time as or after adding the adapter primers. Extension of the annealed adapter and gap-filling primers leads to production of double-stranded adapter amplicons. This amplification cycle is repeated several time, such as 5-30 times, preferably about 15-20 times, to produce a amplify double-stranded adapter amplicons comprising the P5 and the P7 adapter at their respective ends, representing a sequencing library for use in an Illumina sequencing reaction.

For performing step (m) or steps (j) to (m), which can be a regular PCR, additional reagents may be added to the reaction mixture, such as additional DNA polymerase.

It is a great advantage of the method of generating a sequencing library of the present invention that it efficiently generates dsDNA with two different adapter sequences at both ends, which are required for subsequent sequencing reactions, such as NGS using the Illumina technology. There are no dsDNA molecules with two identical adapter sequences at both ends of the dsDNA generated from the method of the invention, so that no additional purification step is required.

In embodiments, the method of generating a sequencing library comprises the provision of amplicons of genomic DNA of more than one samples, such as more than one single cells, wherein the amplicons of each single cell comprise an individual sequence-label, the method comprising
a. providing in a reaction vessel pooled third-round amplicons generated by the method of amplifying DNA of more than one samples of the present invention,
b. adding a 5'-exonuclease to the reaction vessel to produce a reaction mixture,
c. subjecting the reaction mixture to a temperature at which the 5'-exonuclease is active to generate double-stranded third-round amplicons with overhanging single-stranded 3'-ends,
d. adding to the reaction vessel
   - gap-filling primers, wherein the gap-filling primers comprise
      o at their 3'-end a sequence comprised by the sequence at the 3'-end of the second primers and
      o a first adapter sequence for a subsequent sequencing of the amplicons,
   - a DNA polymerase, preferably without strand-displacement (SD) activity, and
   - a ligase,
e. subjecting the reaction mixture to a temperature at which
   - a gap-filling primer hybridizes to the overhanging single-stranded 3'-end of a double-stranded third-round amplicon,
   - the 3'-end of the third primer is extended by the DNA polymerase without SD activity and
   - the extended 3'-end of the gap-filling primer is ligated to the 5'-end of the double-stranded third-round amplicon by the ligase.

In preferred embodiments, the provided third-round amplicons comprise near their 5'-ends one or more modified nucleotides resistant to a 5'-exonuclease, such as nucleotides with a phosphorothioate modification or LNA nucleotides.

In embodiments, the method of generating a sequencing library comprising the provision of third-round amplicons generated by the method of amplifying genomic DNA comprised in more than one samples of the present invention disclosed herein also comprises
f. adding adapter primers to the reaction vessel, wherein the adapter primers comprise
   - at their 3'-end a sequence comprised by the sequence at the 3'-end of the second primers, wherein preferably the sequence at the 3'-end of the adapter primers comprises or consists of the sequence at the 3'-end of the second primers, wherein more preferably the sequence at the 3'-end of the adapter primers comprises or consists of the sequence of the second primers, and
   - a second adapter sequence for a subsequent sequencing of the amplicons,
g. subjecting the reaction mixture to a temperature to produce single stranded amplicons,
h. subjecting the reaction mixture to a temperature at which the adapter primers anneal to the 3'-ends of the single-stranded third-round amplicons comprising ligated gap-filling primers at their 5'-end,
i. subjecting the reaction mixture to a temperature at which the DNA polymerase extends the 3'-ends of the annealed adapter primers and of the single-stranded amplicons comprising ligated gap-filling primers to produce double-stranded adapter amplicons.

In embodiments, the method of generating a sequencing library comprising amplicons of genomic DNA of more than one samples comprising the provision of third-round amplicons disclosed herein also comprises
j. subjecting the reaction mixture to a temperature to produce single stranded adapter amplicons,
k. subjecting the reaction mixture to a temperature at which the adapter primers and gap-filling primers anneal to the 3'-ends of the corresponding single-stranded adapter amplicons,
I. subjecting the reaction mixture to a temperature at which the DNA polymerase extends the 3'-ends of the annealed adapter and gap-filling primers to produce double stranded adapter amplicons,
m. repeating the steps (j) - (I) to produce double-stranded adapter amplicons.

### Embodiments of the invention relating to a kit for performing a method of the invention

The present invention also relates to a kit for amplifying dsDNA of more than one samples, such as more than one single cells, for generating a sequencing library. Preferably, the invention relates to a kit for amplifying the whole genome of more than one samples, such as more than one single cells, for generating a sequencing library. The kit of the invention comprises at least two kinds of first primers,
- wherein each kind of first primers is provided in an individual container,
- wherein each kind of first primer comprises in a 5' to 3' orientation a constant sequence and a variable sequence,
- wherein the constant sequence comprises in a 5' to 3' orientation a first part with a sequence that is common for each of the at least two kinds of first primers, and a second part with a sequence that is different for each of the at least two kinds of first primers.

In embodiments, the kit for generating a sequencing library of the invention comprises at least 12, preferably at least 96, more preferably at least 384 kinds of first primers, wherein the primers are preferably provided in sealed individual wells or a multiwell plate.

In embodiments of the kit of the invention, each kind of first primers comprises in the constant sequence modified nucleotides resistant to a 5'-exonuclease, such as nucleotides with a phosphorothioate modification or LNA nucleotides. In embodiments, at least 1, 2, 3, 4, 5, or 6 nucleotides of the second part of the constant region are modified. It is preferable that the modified nucleotides are located at the 5'-end of the second part of the constant region.

In the context of the kit of the invention, the first primers can be phosphorylated at their 5'-ends. In embodiments of the kit of the invention, each kind of first primers comprises between 10 and 70 nucleotides, preferably about 10-40 nucleotides, wherein the first part of the constant sequence comprises between 5 and 66 nucleotides, preferably 5-36 nucleotides and the second part of the constant sequence comprises between 4 and 10 nucleotides.

In further embodiments, the kit of the invention comprises additionally in an individual container second primers comprising at their 3'-end a sequence comprised by the first part of the constant sequence of the first primers. In embodiments of the kit, the second primers comprise at their 3'-end at least one modified nucleotide, preferably at least 3 to 6 modified nucleotides, resistant to a 5'-exonuclease, such as nucleotides with a phosphorothioate modification or LNA nucleotides. In embodiments, such modified nucleotides are not located at the 3'-end of the second primers but near the 3'-end of the second primers, such as within the first 10, 9, 8, 7, 6, 5, 4, 3 or 2 nucleotides from the 3'-end.

In further embodiments, the second primers are phosphorylated at their 5'-ends.

In embodiments, the kit of the invention comprises additionally gap-filling primers comprising at their 3'-end a sequence comprised by the sequence at the 3'-end of the second primers, wherein the sequence at the 3'-end of the gap-filling primers enables hybridization/annealing of the gap-filling primers to the sequence at the 3'-end of the second primers, and a first adapter sequence for a subsequent sequencing of the amplicons.

In further embodiments, the kit for generating a sequencing library of the invention comprises adapter primers comprising at their 3'-end a sequence comprised by the sequence at the 3'-end of the second primers, wherein the sequence at the 3'-end of the adapter primers enables hybridization/annealing of the adapter primers to the sequence at the 3'-end of the second primers with a higher affinity than the sequence at the 3'-end of the gap-filling primers, and a second adapter sequence for a subsequent sequencing of the amplicons. In embodiments, the sequence at the 3'-end of the adapter primers comprises or consists of the sequence of the second primer.

In embodiments, the kit for generating a sequencing library of the invention comprises one or more enzymes for performing any of the methods of the present invention, such as a polymerase, a polymerase with strand displacement activity, a polymerase without strand displacement activity, a polymerase with 5'-3' exonuclease activity, a polymerase without 5'-3' exonuclease activity, a 5'-exonuclease, such as a lambda exonuclease, and/or a ligase.

The invention further relates to a kit for generating a sequencing library from provided dsDNA, the kit comprising
- gap-filling primers comprising
   o at their 3'-end a sequence that enables hybridization/annealing of the gap-filling primers to a sequence at a 3'-end of the strands of the provided dsDNA, and
   o a first adapter sequence for a subsequent sequencing of the provided dsDNAs, preferably at their 5'-end, and
- adapter primers comprising
   o at their 3'-end a sequence that enables hybridization/annealing of the adapter primers to a sequence at a 3'-end of the strands of the provided dsDNAs, preferably with a higher affinity than the sequence at the 3'-end of the gap-filling primers, and
   o a second adapter sequence for a subsequent sequencing of the provided dsDNA, preferably at their 5'-end,
- and optionally one or more enzymes for performing the method of generating a sequencing library of the invention, such as a polymerase, a polymerase with strand displacement activity, a polymerase without strand displacement activity, a polymerase with 5'-3' exonuclease activity, a polymerase without 5'-3' exonuclease activity, a 5'-exonuclease, such as a lambda exonuclease, and/or a ligase, preferably a 5'-exonuclease.

The features disclosed in the context of one method of the present invention also apply to the other methods and to the kits of the invention and the other way around. For example, if a specific feature of a certain oligonucleotide or primer, such as a feature of a first primer, has been disclosed in the context of the method of amplifying genomic DNA of the invention, this feature also applies to and is herewith disclosed in the context of a kit of the present invention or in the context of the method of generating a sequencing library of the invention.

### DETAILED DESCRIPTION OF THE INVENTION

All cited documents of the patent and non-patent literature are hereby incorporated by reference in their entirety.

The methods and kits of the present invention are in the field of DNA amplification, in particular whole genome amplification, in particular whole genome amplification of single cells. As used herein the term amplifying DNA comprises amplifying genomic DNA and in particular WGA. DNA amplification and WGA in particular are mostly performed on DNA material of samples comprising very little amounts of DNA, such as samples comprising genomic material of one single cell, such as a prokaryotic or eukaryotic cell. Furthermore, DNA amplification can also be performed using DNA of samples that comprise very little amounts of DNA of unknown origin, which are not derived from a single cell. In order to analyze the sequence of DNA material provided in very low quantities in a sample, the provided DNA has to be first amplified before it can be subjected to a sequence analysis reaction, such as a NGS, preferably a sequencing reaction using the predominant Illumina technology. Preferably, the amplification is not creating a bias towards certain sequences in order to ensure that quantitative results can be generated from the sequencing analysis. It is an important advantage of the DNA amplification method of the invention that is a quasilinear reaction that avoids introduction of an amplification bias.

In a particular aspect, embodiments are directed to methods for the amplification of substantially the entire genome or entire transcriptome without loss of representation of specific sites (herein defined as "whole genome amplification" and "whole transcriptome amplification", respectively). In a specific embodiment, whole genome amplification comprises simultaneous amplification of substantially all fragments of a genomic library. In a further specific embodiment, "substantially entire" or "substantially all" refers to about 80%, about 85%, about 90%, about 95%, about 97%, or about 99% of all sequence in a genome. A skilled artisan recognizes that amplification of the whole genome will, in some embodiments, comprise non-equivalent amplification of particular sequences over others.

In one aspect, embodiments of the invention relate to methods of amplifying DNA or generating a sequencing library that can be performed in a single tube or in a micro-titer plate, for example, in a high throughput format.

Single cell sequencing examines the sequence information from individual cells with optimized next generation sequencing (NGS) technologies, providing a higher resolution of cellular differences and a better understanding of the function of an individual cell in the context of its microenvironment. Sequencing the DNA of individual cells can give information about mutations carried by small populations of cells, for example in cancer, sequencing the RNAs expressed by individual cells can give insight into the existence and behavior of different cell types, for example in development. Single cell DNA genome sequencing involves isolating a single cell, performing whole-genome-amplification (WGA), constructing sequencing libraries and then sequencing the DNA using a next-generation sequencer, for example an Illumina sequencer.

The DNA amplified by the method of the invention can be sequenced and analyzed using methods known to those of skill in the art. Determination of the sequence of a nucleic acid sequence of interest can be performed using a variety of sequencing methods known in the art including, but not limited to, sequencing by hybridization (SBH), sequencing by ligation (SBL) (Shendure et al. (2005) Science 309: 1728), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads (U.S. Pat. No. 7,425,431), wobble sequencing (PCT US05/27695), multiplex sequencing (U.S. Serial No. 12/027,039, filed February 6, 2008; Porreca et al (2007) Nat. Methods 4:931), polymerized colony (POLONY) sequencing (U.S. Patent Nos. 6,432,360, 6,485,944 and 6,51 1 ,803, and PCT/US05/06425); nanogrid rolling circle sequencing (ROLONY) (U.S. Serial No. 12/120,541, filed May 14, 2008), allele- specific oligo ligation assays (e.g., oligo ligation assay (OLA), single template molecule OLA using a ligated linear probe and a rolling circle amplification (RCA) readout, ligated padlock probes, and/or single template molecule OLA using a ligated circular padlock probe and a rolling circle amplification (RCA) readout) and the like. High-throughput sequencing methods, e.g., using platforms such as Roche 454, Illumina Solexa, AB-SOLiD, Helicos, Polonator platforms and the like, can also be utilized. A variety of light-based sequencing technologies are known in the art (Landegren et al. (1998) Genome Res. 8:769-76; Kwok (2000) Pharmacogenomics 1 :95-100; and Shi (2001) Clin. Chem. 47: 164-172).

NGS sequencing using the Illumina technology works in three basic steps: amplify, sequence, and analyze. The process begins with provision of DNA, purified DNA or purified DNA fragments. The DNA gets fragmented up into smaller pieces of mostly less than 1000 nucleotides/base pairs and given adapters, potentially barcoding-sequences and other kinds of molecular modifications that act as reference points during amplification, sequencing, and analysis are added. The modified DNA is loaded onto a specialized chip where amplification and sequencing will take place. Along the bottom of the chip are hundreds of thousands of oligonucleotides (short, synthetic pieces of DNA). They are anchored to the chip and able to grab DNA fragments that have complementary adapter sequences. Once the fragments have attached, cluster generation begins. Cluster generation results in about a thousand copies of each fragment of DNA. Next, primers and modified nucleotides enter the chip and these nucleotides have reversible 3' blockers that force the polymerase to add on only one nucleotide at a time as well as fluorescent tags. After each round of synthesis, a camera takes a picture of the chip. A computer determines what base was added by the wavelength of the fluorescent tag and records it for every spot on the chip. After each round, non-incorporated molecules are washed away. A chemical deblocking step is then used in the removal of the 3' terminal blocking group and the dye in a single step. The process continues until the full DNA molecule is sequenced. With this technology, thousands of places throughout the genome are sequenced at once via massive parallel sequencing.

Accordingly, for Illumina sequencing, as for other sequencing technologies, it is required to provide purified double-stranded DNA fragments with a length of not more than 1000 nucleotides and suitable adapter sequences at both ends. As used herein, a method that modifies one or dsDNA molecules, preferably multiple dsDNA fragments or amplicons, that are to be subjected to a NGS reaction, preferably using the Illumina technology, in a way that dsDNA molecules of mostly no more than 1000 bp having two different adapter sequences attached to both of their ends is referred to as a "method of generating a sequencing library". Usual methods of generating a sequencing library from sample comprising very small amounts of DNA, such as samples comprising genomic DNA of a single cell, involve fragmentation of the amplicons of genomic DNA generated from WGA, since WGA method of the state of the art generate amplicons that are mostly longer than 1000 bp and do not generate a sufficient number of amplicons of not more than 1000 bp in order to cover the whole DNA provided in a sample, such as the whole genome of a mammalian or human cell. Therefore, amplicons of genomic DNA generated by WGA have to be fragmented prior to ligation or introduction of adapter sequences, which may comprise a barcoding-sequence for identifying DNA material originating from a specific sample. Therefore, pooling of DNA material from different samples can only occur after library preparation, when using methods of the state of the art.

The term "dsDNA molecule" refers to a dsDNA composed of two complementary strands of DNA that are bound to each other via base-pairing. Although a dsDNA molecules is composed of two individual DNA molecules, the term as used herein refers to the hybridized complex of two DNA strands.

According to one aspect, the method of amplifying DNA further includes genotype analysis of the amplified DNA product. Alternatively, the method of amplifying DNA preferably further includes identifying a polymorphism such as a single nucleotide polymorphism (SNP) in the amplified DNA product. In preferred embodiments, a SNP may be identified in the DNA of an organism by a number of methods well known to those of skill in the art, including but not limited to identifying the SNP by DNA sequencing, by amplifying a PCR product and sequencing the PCR product, by Oligonucleotide Ligation Assay (OLA), by Doublecode OLA, by Single Base Extension Assay, by allele specific primer extension, or by mismatch hybridization. Preferably the identified SNP is associated with a phenotype, including disease phenotypes and desirable phenotypic traits. The amplified DNA generated by using the disclosed method of DNA amplification may also preferably be used to generate a DNA library, including but not limited to genomic DNA libraries, microdissected chromosome DNA libraries, BAC libraries, YAC libraries, PAC libraries, cDNA libraries, phage libraries, and cosmid libraries.

The term "genome" as used herein is defined as the collective gene set carried by an individual, cell, or organelle. The term "genomic DNA" as used herein is defined as DNA material comprising the partial or full collective gene set carried by an individual, cell, or organelle. The term "transcriptome" as used herein is defined as the collective RNA set expressed within a cell. As used herein, the term "nucleoside" refers to a molecule having a purine or pyrimidine base covalently linked to a ribose or deoxyribose sugar. Exemplary nucleosides include adenosine, guanosine, cytidine, uridine and thymidine. Additional exemplary nucleosides include inosine, 1 - methyl inosine, pseudouridine, 5,6-dihydrouridine, ribothymidine, ²N-methylguanosine and ^{2'2}N,N-dimethylguanosine (also referred to as "rare" nucleosides). The term "nucleotide" refers to a nucleoside having one or more phosphate groups joined in ester linkages to the sugar moiety. Exemplary nucleotides include nucleoside monophosphates, diphosphates and triphosphates. The terms "polynucleotide," "oligonucleotide" and "nucleic acid molecule" are used interchangeably herein and refer to a polymer of nucleotides, either deoxyribonucleotides or ribonucleotides, of any length joined together by a phosphodiester linkage between 5' and 3' carbon atoms. Polynucleotides can have any three-dimensional structure and can perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. The term also refers to both double- and single-stranded molecules. Unless otherwise specified or required, any embodiment of this invention that comprises a polynucleotide encompasses both the double-stranded form and each of two complementary single-stranded forms known or predicted to make up the double-stranded form. A polynucleotide is composed of a specific sequence of four nucleotide bases: adenine (A); cytosine (C); guanine (G); thymine (T); and uracil (U) for thymine when the polynucleotide is RNA. Thus, the term polynucleotide sequence is the alphabetical representation of a polynucleotide molecule. This alphabetical representation can be input into databases in a computer having a central processing unit and used for bioinformatics applications such as functional genomics and homology searching. The terms "RNA," "RNA molecule" and "ribonucleic acid molecule" refer to a polymer of ribonucleotides. The terms "DNA," "DNA molecule" and "deoxyribonucleic acid molecule" refer to a polymer of deoxyribonucleotides. DNA and RNA can be synthesized naturally (e.g., by DNA replication or transcription of DNA, respectively). RNA can be post-transcriptionally modified. DNA and RNA can also be chemically synthesized. DNA and RNA can be single-stranded (i.e., ssRNA and ssDNA, respectively) or multi-stranded (e.g., double stranded, i.e., dsRNA and dsDNA, respectively). "mRNA" or "messenger RNA" is single-stranded RNA that specifies the amino acid sequence of one or more polypeptide chains. This information is translated during protein synthesis when ribosomes bind to the mRNA.

The terms "nucleotide analog," "altered nucleotide" and "modified nucleotide" refer to a non-standard nucleotide, including non-naturally occurring ribonucleotides or deoxyribonucleotides. In certain exemplary embodiments, nucleotide analogs are modified at any position so as to alter certain chemical properties of the nucleotide yet retain the ability of the nucleotide analog to perform its intended function. Examples of positions of the nucleotide which may be derivitized include the 5 position, e.g., 5-(2-amino)propyl uridine, 5-bromo uridine, 5-propyne uridine, 5-propenyl uridine, etc.; the 6 position, e.g., 6-(2- amino) propyl uridine; the 8-position for adenosine and/or guanosines, e.g., 8- bromo guanosine, 8-chloro guanosine, 8-fluoroguanosine, etc. Nucleotide analogs also include deaza nucleotides, e.g., 7-deaza-adenosine; O- and N-modified (e.g., alkylated, e.g., N6-methyl adenosine, or as otherwise known in the art) nucleotides; and other heterocyclically modified nucleotide analogs such as those described in Herdewijn, Antisense Nucleic Acid Drug Dev., 2000 Aug. 10(4):297-310. Nucleotide analogs may also comprise modifications to the sugar portion of the nucleotides. For example the 2' OH-group may be replaced by a group selected from H, OR, R, F, CI, Br, I, SH, SR, NH₂, NHR, NR₂, COOR, or OR, wherein R is substituted or unsubstituted C1 -C6 alkyl, alkenyl, alkynyl, aryl, etc.Other possible modifications include those described in U.S. Pat. Nos. 5,858,988, and 6,291,438.

In the method of generating a sequencing library of the present invention, a 5'-exonuclease is used in order to generate free 3'-ends of dsDNA molecules. Any 5'-exonuclease known to a skilled person can be used in the context of the invention. In order to prevent removal of a barcoding-sequence comprised in a dsDNA molecules that is subjected to the library generation method of the invention, one or both strands of the provided dsDNA molecules, such as third-round amplicons generated from the method of amplifying DNA of the present invention, comprise modified nucleotides resistant to a 5'-exonuclease near their 5'-end. In the context of the invention, modified nucleotides resistant to a 5'-exonuclease are understood as nucleotides that have been modified so they cannot be removed by a 5'-exonuclease. Accordingly, incorporation of modified nucleotides resistant to a 5'-exonuclease into a nucleotide sequence leads to a stop of the continuous hydrolysis reaction degrading the nucleotide sequence of the DNA strand from the 5'-end. Modified nucleotides resistant to a 5'-exonuclease include nucleotides with a phosphorothioate modification or LNA nucleotides.

The modification of DNA by phosphorothioate (PT) occurs when the non-bridging oxygen in the sugar-phosphate backbone of DNA is replaced with sulfur. This modification renders the internucleotide linkage resistant to nuclease degradation. Phosphorothioate bonds can be introduced between the last 3-10, preferably 3-5 nucleotides at the 5'- or 3'-end of purchasable oligonucleotides to inhibit exonuclease degradation.

LNA stands for locked nucleic acid and is often referred to as inaccessible RNA. It is a modified RNA nucleotide in which the ribose moiety is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired and hybridize with DNA or RNA according to Watson-Crick base-pairing rules.

As used herein, the terms "complementary" and "complementarity" are used in reference to nucleotide sequences related by the base-pairing rules. For example, the sequence 5'-AGT-3' is complementary to the sequence 5'-ACT-3'. Complementarity can be partial or total. Partial complementarity occurs when one or more nucleic acid bases is not matched according to the base pairing rules. Total or complete complementarity between nucleic acids occurs when each and every nucleic acid base is matched with another base under the base pairing rules. The degree of complementarity between nucleic acid strands has significant effects on the efficiency and strength of hybridization between nucleic acid strands. The term "homology" when used in relation to nucleic acids refers to a degree of complementarity. There may be partial homology (i.e., partial identity) or complete homology (i.e., complete identity). A partially complementary sequence is one that at least partially inhibits a completely complementary sequence from hybridizing to a target nucleic acid and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (i.e., an oligonucleotide which is capable of hybridizing to another oligonucleotide of interest) will compete for and inhibit the binding (i.e., the hybridization) of a completely homologous sequence to a target under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target which lacks even a partial degree of complementarity (e.g., less than about 30% identity); in the absence of non-specific binding the probe will not hybridize to the second non-complementary target. When used in reference to a double-stranded nucleic acid sequence such as a cDNA or genomic clone, the term "substantially homologous" refers to any probe or primer or oligonucleotide which can hybridize to either or both strands of the double-stranded nucleic acid sequence under conditions of low stringency. When used in reference to a single- stranded nucleic acid sequence, the term "substantially homologous" refers to any probe which can hybridize to the single-stranded nucleic acid sequence under conditions of low stringency.

The following terms are used to describe the sequence relationships between two or more polynucleotides: "reference sequence," "sequence identity," "percentage of sequence identity" and "substantial identity". A "reference sequence" is a defined sequence used as a basis for a sequence comparison; a reference sequence may be a subset of a larger sequence, for example, as a segment of a full-length cDNA sequence given in a sequence listing or may comprise a complete gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (Smith and Waterman (1981) Adv. Appl. Math. 2:482) by the homology alignment algorithm of Needleman and Wunsch (J. Mol. Biol. 48:443 (1970)), by the search for similarity method of Pearson and Lipman (Proc. Natl. Acad Sci. USA 85:2444 (1988)]), by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (i.e., resulting in the highest percentage of homology over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The term "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence, for example, as a segment of the full-length sequences of the compositions claimed in the present invention.

The term "hybridization" refers to the pairing of complementary nucleic acids. Hybridization and the strength of hybridization (i.e., the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

The term "melting temperature" or "Tₘ" refers to the temperature at which a double stranded nucleic acid melt or dehybridizes. The melting temperature is the temperature at which a population of double-stranded nucleic acid molecules becomes half dissociated into single strands. The equation for calculating the Tₘ of nucleic acids is well known in the art. As indicated by , standard references, a simple estimate of the Tₘ value may be calculated by the equation: Tₘ = 81.5 + 0.41 (% G + C), when a nucleic acid is in aqueous solution at 1 M NaCl (See, e.g., Anderson and Young, Quantitative Filter Hybridization, in Nucleic Acid Hybridization (1985)). Other references include more sophisticated computations that take structural as well as sequence characteristics into account for the calculation of Tₘ.

In embodiments, the present invention relates to the amplification and optionally subsequent analysis of genomic DNA of a single cell. In certain exemplary embodiments, cells are identified and then a single cell or a plurality of cells are isolated. Cells within the scope of the present disclosure include any type of cell where understanding the DNA or RNA content is considered by those of skill in the art to be useful. A cell according to the present disclosure includes a cancer cell of any type, hepatocyte, oocyte, embryo, stem cell, iPS cell, ES cell, neuron, erythrocyte, melanocyte, astrocyte, germ cell, oligodendrocyte, kidney cell and the like. According to one aspect, the methods of the present invention are practiced with the cellular RNA or cellular DNA from a single cell. The methods of the invention relate to the analysis of more than one sample, preferably more than one cell. This means, that the methods of the invention relate to the analysis of a plurality of samples, preferably cells. A plurality of cells includes from about 2 to about 1,000,000 cells, about 2 to about 10 cells, about 2 to about 100 cells, about 2 to about 1,000 cells, about 2 to about 10,000 cells, about 2 to about 100,000 cells, about 2 to about 10 cells or about 2 to about 5 cells.

Nucleic acids processed by methods described herein may be DNA, RNA, or DNA-RNA chimeras, and they may be obtained from any useful source, such as, for example, a human sample. In specific embodiments, a double stranded DNA molecule is further defined as comprising a genome, such as, for example, one obtained from a sample from a human. The sample may be any sample from a human, such as blood, serum, plasma, cerebrospinal fluid, cheek scrapings, nipple aspirate, biopsy, semen (which may be referred to as ejaculate), urine, feces, hair follicle, saliva, sweat, immunoprecipitated or physically isolated chromatin, and so forth. In specific embodiments, the sample comprises a single cell. In particular embodiments, the amplified nucleic acid molecule from the sample provides diagnostic or prognostic information. For example, the prepared nucleic acid molecule from the sample may provide genomic copy number and/or sequence information, allelic variation information, cancer diagnosis, prenatal diagnosis, paternity information, disease diagnosis, detection, monitoring, and/or treatment information, sequence information, and so forth. As used herein, a "single cell" refers to one cell. Single cells useful in the methods described herein can be obtained from a tissue of interest, or from a biopsy, blood sample, or cell culture. Additionally, cells from specific organs, tissues, tumors, neoplasms, or the like can be obtained and used in the methods described herein. Furthermore, in general, cells from any population can be used in the methods, such as a population of prokaryotic or eukaryotic single celled organisms including bacteria or yeast. A single cell suspension can be obtained using standard methods known in the art including, for example, enzymatically using trypsin or papain to digest proteins connecting cells in tissue samples or releasing adherent cells in culture, or mechanically separating cells in a sample. Single cells can be placed in any suitable reaction vessel in which single cells can be treated individually. For example, a 96-well plate, such that each single cell is placed in a single well. Methods for manipulating single cells are known in the art and include fluorescence activated cell sorting (FACS), single-cell dispensing (Riba et al., "Molecular Genetic Characterization of Individual Cancer Cells Isolated via Single-Cell Printing," PLoS One, vol. 11, no. 9, p. 10.1371/journal.pone.0163455, 2016), flow cytometry (Herzenberg., PNAS USA 76: 1453-55 1979), micromanipulation and the use of semi-automated cell pickers (e.g. the Quixell™ cell transfer system from Stoelting Co.). Individual cells can, for example, be individually selected based on features detectable by microscopic observation, such as location, morphology, or reporter gene expression. Additionally, a combination of gradient centrifugation and flow cytometry can also be used to increase isolation or sorting efficiency.

Once a desired cell has been identified, the cell can be lysed to release cellular contents including DNA and RNA, using methods known to those of skill in the art. The cellular contents are contained within a vessel. In some aspects of the invention, cellular contents, such as genomic DNA and RNA, can be released from the cells by lysing the cells. Lysis can be achieved by, for example, heating the cells, or by the use of detergents or other chemical methods, or by a combination of these. However, any suitable lysis method known in the art can be used. A mild lysis procedure can advantageously be used to prevent the release of nuclear chromatin. For example, heating the cells at 72°C for 2 minutes in the presence of Tween-20 is sufficient to lyse the cells while resulting in no detectable genomic contamination from nuclear chromatin. Alternatively, cells can be heated to 65°C for 10 minutes in water (Esumi et al., Neurosci Res 60(4):439-51 (2008)); or 70°C for 90 seconds in PCR buffer II (Applied Biosystems) supplemented with 0.5% NP-40 (Kurimoto et al., Nucleic Acids Res 34(5):e42 (2006)); or lysis can be achieved with a protease such as Proteinase K or by the use of chaotropic salts such as guanidine isothiocyanate (U.S. Publication No. 2007/0281313). Amplification of genomic DNA according to methods described herein can be performed directly on cell lysates, such that a reaction mix can be added to the cell lysates. Alternatively, the cell lysate can be separated into two or more volumes such as into two or more containers, tubes or regions using methods described herein or methods known to those of skill in the art with a portion of the cell lysate contained in each volume container, tube or region. Genomic DNA contained in each container, tube or region may then be amplified by methods described herein or methods known to those of skill in the art. A nucleic acid used in the invention can also include native or non-native bases. In this regard a native deoxyribonucleic acid can have one or more bases selected from the group consisting of adenine, thymine, eytosine or guanine and a ribonucleic acid can have one or more bases selected from the group consisting of uracil, adenine, cytosine or guanine. Exemplary non-native bases that can be included in a nucleic acid, whether having a native backbone or analog structure, include, without limitation, inosine, xathanine, hypoxathanine, isocytosine, isoguanine, 5-methylcytosine, 5 -hydroxy methyl cytosine, 2-aminoadenine, 6- methyl adenine, 6-methyl guanine, 2-propyl guanine, 2-propyl adenine, 2- thioLiracil, 2-thiothymine, 2- thiocytosine, 15 - halouracil, 15 -halocytosine, 5- propynyl uracil, 5-propynyl cytosine, 6-azo uracil, 6-azo cytosine, 6-azo thymine, 5-uracil, 4-thiouracil, 8-halo adenine or guanine, 8- amino adenine or guanine, 8-thiol adenine or guanine, 8-thioalkyl adenine or guanine, 8- hydroxyl adenine or guanine, 5-halo substituted uracil or cytosine, 7-methylguanine, 7- methyladenine, 8-azaguanine, 8-azaadenine, 7-deazaguanine, 7-deazaadenine, 3-deazaguanine, 3- deazaadenine or the like. A particular embodiment can utilize isocytosine and isoguanine in a nucleic acid in order to reduce non-specific hybridization, as generally described in U.S. Pat. No.5,681 ,702.

As used herein, the term "primer" generally includes an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process are determined by the sequence of the template polynucleotide. Usually primers are extended by a DNA polymerase. Primers usually have a length in the range of between 3 to 36 nucleotides, also 5 to 24 nucleotides, also from 14 to 36 nucleotides. Primers within the scope of the invention include orthogonal primers, amplification primers, constructions primers and the like. Pairs of primers can flank a sequence of interest or a set of sequences of interest. Primers and probes can be degenerate or quasi-degenerate in sequence. Primers within the scope of the present invention bind adjacent to a target sequence. A "primer" may be considered a short polynucleotide, generally with a free 3' -OH group that binds to a target or template potentially present in a sample of interest by hybridizing with the target, and thereafter promoting polymerization of a polynucleotide complementary to the target. Primers of the instant invention are comprised of nucleotides ranging from 17 to 30 nucleotides. In one aspect, the primer is at least 17 nucleotides, or alternatively, at least 18 nucleotides, or alternatively, at least 19 nucleotides, or alternatively, at least 20 nucleotides, or alternatively, at least 21 nucleotides, or alternatively, at least 22 nucleotides, or alternatively, at least 23 nucleotides, or alternatively, at least 24 nucleotides, or alternatively, at least 25 nucleotides, or alternatively, at least 26 nucleotides, or alternatively, at least 27 nucleotides, or alternatively, at least 28 nucleotides, or alternatively, at least 29 nucleotides, or alternatively, at least 30 nucleotides, or alternatively at least 50 nucleotides, or alternatively at least 75 nucleotides or alternatively at least 100 nucleotides.

DNA amplified in a first step using the methods describe herein can be further amplified using methods known to those of skill in the art. In certain aspects, amplification is achieved using PCR. The term "polymerase chain reaction" ("PCR") of Mullis (U.S. Pat. Nos. 4,683, 195, 4,683,202, and 4,965, 188) refers to a method for increasing the concentration of a segment of a target sequence in a mixture of nucleic acid sequences without cloning or purification. This process for amplifying the target sequence consists of introducing a large excess of two oligonucleotide primers to the nucleic acid sequence mixture containing the desired target sequence, followed by a precise sequence of thermal cycling in the presence of a polymerase (e.g., DNA polymerase). The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing and extension constitute one "cycle;" there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified." With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications. Methods and kits for performing PCR are well known in the art. PCR is a reaction in which replicate copies are made of a target polynucleotide using a pair of primers or a set of primers consisting of an upstream and a downstream primer, and a catalyst of polymerization, such as a DNA polymerase, and typically a thermally-stable polymerase enzyme. Methods for PCR are well known in the art, and taught, for example in MacPherson et al. (1991) PCR 1 : A Practical Approach (IRL Press at Oxford University Press). All processes of producing replicate copies of a polynucleotide, such as PCR or gene cloning, are collectively referred to herein as replication. A primer can also be used as a probe in hybridization reactions, such as Southern or Northern blot analyses. The expression "amplification" or "amplifying" refers to a process by which extra or multiple copies of a particular polynucleotide are formed. Amplification includes methods such as PCR, ligation amplification (or ligase chain reaction, LCR) and amplification methods. These methods are known and widely practiced in the art. See, e.g., U.S. Patent Nos. 4,683,195 and 4,683,202 and Innis et al., "PCR protocols: a guide to method and applications" Academic Press, Incorporated (1990) (for PCR); and Wu et al. (1989) Genomics 4:560-569 (for LCR). In general, the PCR procedure describes a method of gene amplification which is comprised of (i) sequence-specific hybridization of primers to specific genes within a DNA sample (or library), (ii) subsequent amplification involving multiple rounds of annealing, elongation, and denaturation using a DNA polymerase, and (iii) screening the PCR products for a band of the correct size. The primers used are oligonucleotides of sufficient length and appropriate sequence to provide initiation of polymerization, i.e. each primer is specifically designed to be complementary to each strand of the genomic locus to be amplified. Reagents and hardware for conducting amplification reaction are commercially available. Primers useful to amplify sequences from a particular gene region are preferably complementary to, and hybridize specifically to sequences in the target region or in its flanking regions and can he prepared using the polynucleotide sequences provided herein. Nucleic acid sequences generated by amplification can be sequenced directly. When hybridization occurs in an antiparallel configuration between two single- stranded polynucleotides, the reaction is called "annealing" and those polynucleotides are described as "complementary". A double-stranded polynucleotide can be complementary or homologous to another polynucleotide, if hybridization can occur between one of the strands of the first polynucleotide and the second. Complementarity or homology (the degree that one polynucleotide is complementary with another) is quantifiable in terms of the proportion of bases in opposing strands that are expected to form hydrogen bonding with each other, according to generally accepted base-pairing rules.

The terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences. The term "amplification reagents" refers to those reagents (deoxyribonucleotide triphosphates, buffer, etc.), needed for amplification except for primers, nucleic acid template, and the amplification enzyme. Typically, amplification reagents along with other reaction components are placed and contained in a reaction vessel (test tube, microwell, etc.). Amplification methods include PCR methods known to those of skill in the art and also include rolling circle amplification (Blanco et al., J. Biol. Chem., 264, 8935-8940, 1989), hyperbranched rolling circle amplification (Lizard et al., Nat. Genetics, 19, 225-232, 1998), and loop- mediated isothermal amplification (Notomi et al., Nuc. Acids Res., 28, e63, 2000) each of which are hereby incorporated by reference in their entireties.

"Identity," "homology" or "similarity" are used interchangeably and refer to the sequence similarity between two nucleic acid molecules. Identity can be determined by comparing a position in each sequence which can be aligned for purposes of comparison. When a position in the compared sequence is occupied by the same base or amino acid, then the molecules are homologous at that position. A degree of identity between sequences is a function of the number of matching or identical positions shared by the sequences. An unrelated or nonhomologous sequence shares less than 40% identity, or alternatively less than 25% identity, with one of the sequences of the present invention. A polynucleotide has a certain percentage (for example, 60%, 65%, 70%, 75%, 80%, 85%, 90%, 95%, 98% or 99%) of "sequence identity" to another sequence means that, when aligned, that percentage of bases are the same in comparing the two sequences. This alignment and the percent sequence identity or homology can be determined using software programs known in the art, for example those described in Ausubel et al., Current Protocols in Molecular Biology, John Wiley & Sons, New York, N.Y., (1993). Preferably, default parameters are used for alignment. One alignment program is BLAST, using default parameters. In particular, programs are BLASTN and BLASTP, using the following default parameters: Genetic code = standard; filter = none; strand = both; cutoff- 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the National Center for Biotechnology Information.

The practice of certain embodiments or features of certain embodiments may employ, unless otherwise indicated, conventional techniques of molecular biology, microbiology, recombinant DNA, and so forth which are within ordinary skill in the art. Such techniques are explained fully in the literature. See e.g., Sambrook, Fritsch, and Maniatis, MOLECULAR CLONING: A LABORATORY MANUAL, Second Edition (1989), OLIGONUCLEOTIDE SYNTHESIS (M. J. Gait Ed., 1984), ANIMAL CELL CULTURE (R. I. Freshney, Ed., 1987), the series METHODS IN ENZYMOLOGY (Academic Press, Inc.); GENE TRANSFER VECTORS FOR MAMMALIAN CELLS (J. M. Miller and M. P. Calos eds. 1987), HANDBOOK OF EXPERIMENTAL IMMUNOLOGY, (D. M. Weir and C. C. Blackwell, Eds.), CURRENT PROTOCOLS IN MOLECULAR BIOLOGY (F. M. Ausubel, R. Brent, R. E. Kingston, D. D. Moore, J. G. Siedman, J. A. Smith, and K. Struhl, eds., 1987), CURRENT PROTOCOLS IN IMMUNOLOGY (J. E. coligan, A. M. Kruisbeek, D. H. Margulies, E. M. Shevach and W. Strober, eds., 1991); ANNUAL REVIEW OF IMMUNOLOGY; as well as monographs in journals such as ADVANCES IN IMMUNOLOGY. The skilled person is able to identify on the basis of the listed publications and updated editions thereof suitable techniques to be used in the context of the invention.

Terms and symbols of nucleic acid chemistry, biochemistry, genetics, and molecular biology used herein follow those of standard treatises and texts in the field, e.g., Komberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); and the like.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention, but represent preferred embodiments of aspects of the invention provided for greater illustration of the invention described herein.

### Brief description of the figures:

**Figure 1****:** Overview of the workflow implementing the method of the present invention.
**Figure 2****:** Overview of the first amplification reaction of the method of amplifying genomic DNA of the invention.
**Figure 3****:** Overview of the second amplification reaction of the method of amplifying genomic DNA of the invention.
**Figure 4****:** Overview of the method of preparing a sequencing library of the invention using third-round amplicons from the method of amplifying DNA of the invention.
**Figure 5****:** Single-cell barcoding WGA; pooling of 3 cells.
**Figure 6****:** Single-cell barcoding WGA; PTC; 30 pg gDNA.
**Figure 7****:** Single-cell barcoding WGA; NTC.
**Figure 8****:** Single-cell barcoding WGA; regular MALBAC PCR after quasilinear Amplification

### Detailed description of the figures:

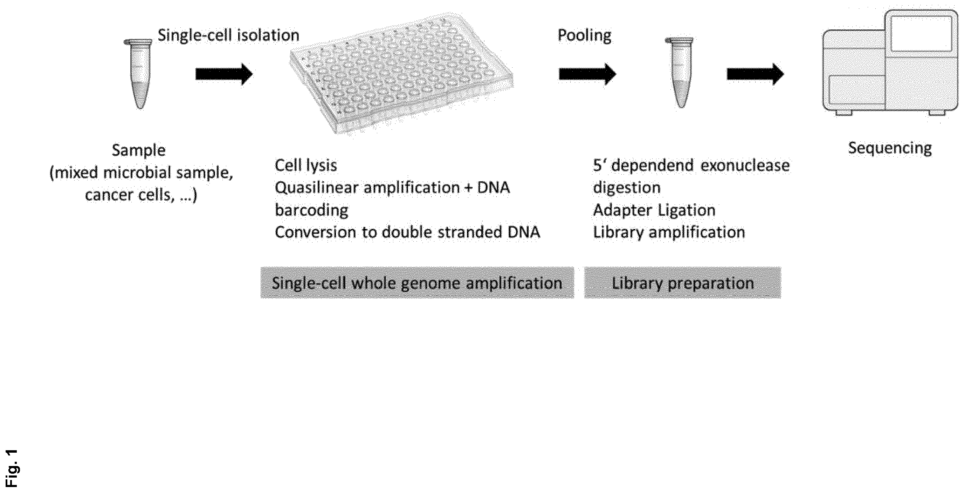

**Figure 1:** Figure 1 shows an overview of the efficient workflow for highly parallel genome analysis of individual cells, which is made possible by the invention. Previously, such an analysis required a library preparation for each individual reaction (i.e. for each individual cell). The workflow outlined here allows for the first time to apply DNA barcodes to the single cell DNA directly during whole genome amplification. This enables pooling of the single cell amplification reaction before ligation of adapter sequences in a single reaction for all cells for library preparation. This enables the cost-intensive library preparation for a large number of cells to be carried out in a single adapter ligation reaction.

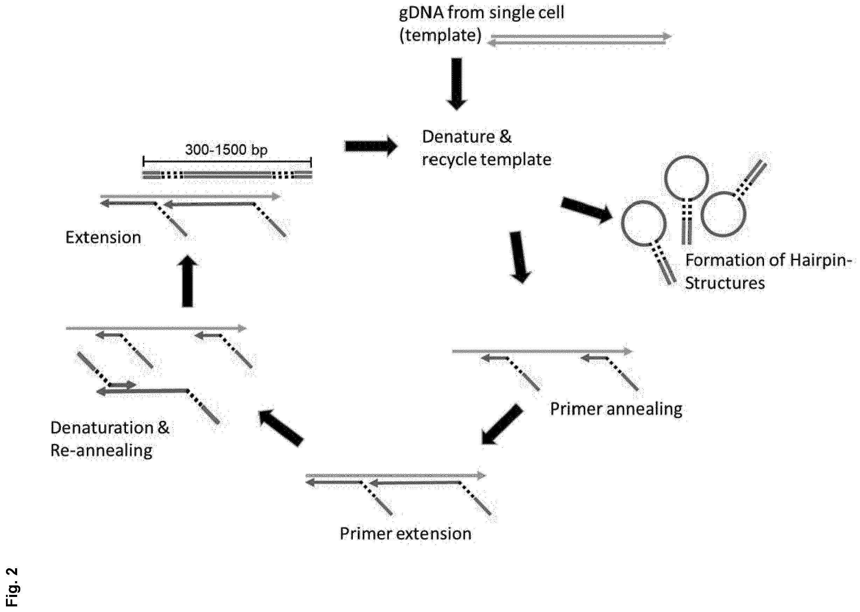

**Figure 2****:** After the lysis of a single cell, specially developed first primers and other components necessary for the amplification reaction (dNTPs, salt ions, polymerase, water) are added to the reaction mixture. The first primers depicted here consist of a variable sequence at the 3'-end of the primers, a reaction-specific barcoding-sequence, and a constant sequence at the 5'-end of the primers. In the depicted embodiments, the constant sequence and the barcoding-sequence consists exclusively of G, A and T nucleotides.

Using the variable sequence, the primer hybridizes at a random location to the genomic DNA. The cell-specific barcoding-sequence is used to clearly mark the amplicons generated from a sample. In the second amplification cycle, self-complementary sequences are formed at the ends of the second-round amplicons, which enable the formation of hairpin structures, which protects already copied sequences from further amplification (quasi-linear amplification).

In addition, the nucleotides of the barcoding-sequence can comprise several phosphorothioate modifications (alternatively LNA bases) to protect the amplicons against degradation by an exonuclease used during the subsequent library preparation method of the invention. The difference to methods known in the art lies in the use of the specially developed primer comprising a barcoding-sequence and potentially nucleotide modification for protection against exonuclease-mediated degradation.

This first amplification reaction enables a quasilinear pre-amplification and can be performed with an strand displacement polymerase. The generated second-round amplicons are protected from further amplification due to hairpin structure formation preventing the molecules from serving as template molecules during the amplification reaction.

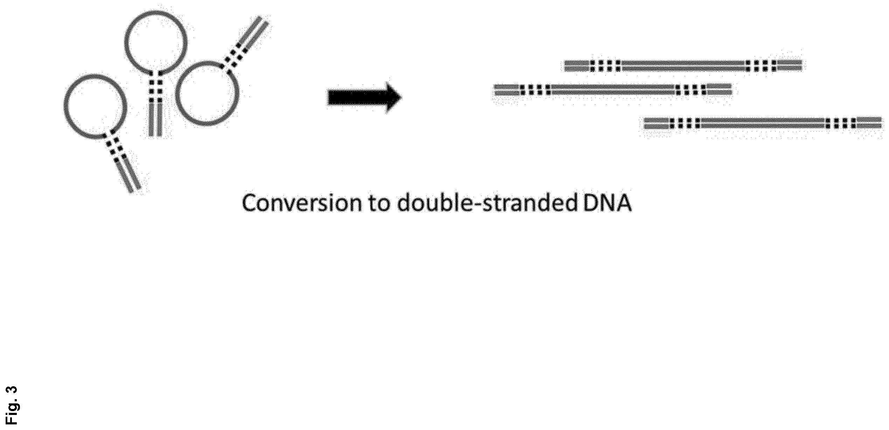

**Figure 3****:** Before the second amplification reaction of the amplification method of the invention is started, second primers are added to the reaction. The second primers contain the distal end of the first primers used in the first amplification reaction of the method of the invention. In embodiments, the second primers also contain several phosphorothioate modifications at the 3'-end. The second primers do not contain the cell-specific barcoding-sequence. This makes it possible to use a universal primer for all reactions in this step. Accordingly, it is possible to pool all reaction mixtures of the first amplification reaction corresponding to individual samples, such as individual cells, as a variant of the method of the invention.

In order to introduce the sequencing adapters asymmetrically to the amplification products in the subsequent method of preparing a sequencing library from the generated amplicons, double-stranded DNA is necessary. In order to convert the primary amplification products (second-round amplicons), which are present in form of hairpins, into double stranded products, these are first denatured at 95°C. Then the annealing of the second primers takes place as in a normal PCR. The added primers compete with the self-complementary ends of the primary hairpin products for binding to the 3' end of the denatured DNA (a balance is formed between the renewed formation of hairpin structures and the binding of the primer). All denatured hairpin structures to which a primer has successfully bound are subsequently converted into double stranded DNA. In the following 6 cycles, the denaturing temperature is chosen so high that already formed double stranded DNA molecules do not denature anymore (in the current protocol 85° C) and thus the hairpin DNA is completely converted into double stranded DNA.

In embodiments, the individual reactions for the individual samples or cells are brought together/pooled after the second amplification reaction of the method of the invention. The amplicons of 10-10000, preferably 48, 96, or 384, such reactions can be combined. The pooling can be followed by a purification step, wherein the combined amplicons are purified, e.g. using functionalized magnetic beads, from the other reagents contained in the reaction mixtures.

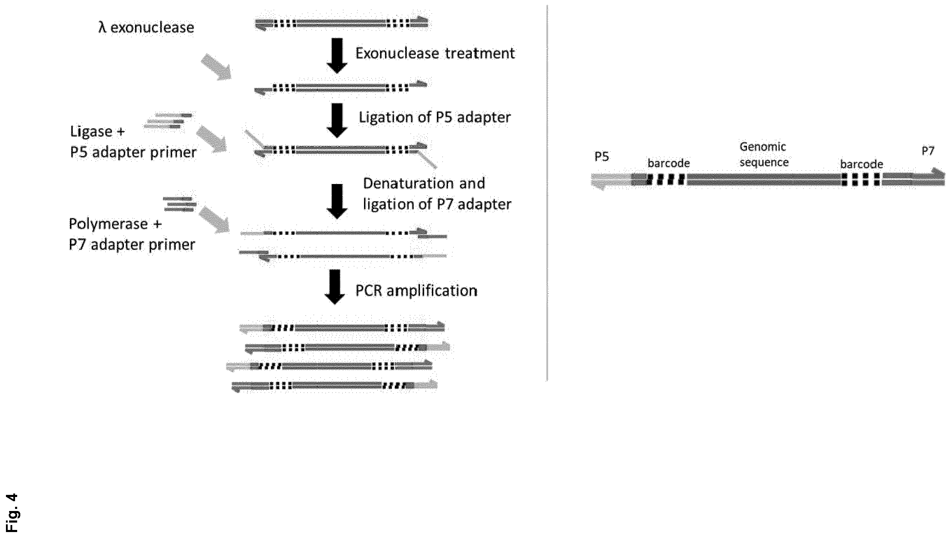

**Figure 4****:** By adding a 5'-exonuclease (e.g. lambda exonuklease) and a suitable reaction buffer, the nucleotides at the 5' ends of the double-stranded DNA are selectively degraded. If lambda exonuclease is used, the used primers have phosphate groups at their 5' ends, which allow a selective and efficient degradation of the 5' ends. Since the barcoding-sequence is protected by phosphorothioate bonds, degradation is stopped at this point (see Figure 3). In the next step, another primer (gap-filling primer) is added which contains the 5'-sided sequencing adapter. This primer is designed to hybridize to the single-stranded part (3' ends) of the amplicons. By adding a polymerase without strand-displacement activity (e.g. Taq polymerase) and a ligase (e.g. ampligase), the hybridized primer can be fully incorporated into the amplicons. This results in the desired asymmetric Y-structure. Subsequently, a so-called adapter primer containing the 3'-sided sequencing adapter is added. The amplicons equipped with the adapters are then amplified by PCR. The fragments are now available as double-stranded DNA with P5 and P7 adapters at each end. After further purification, the sequencing can be started directly.

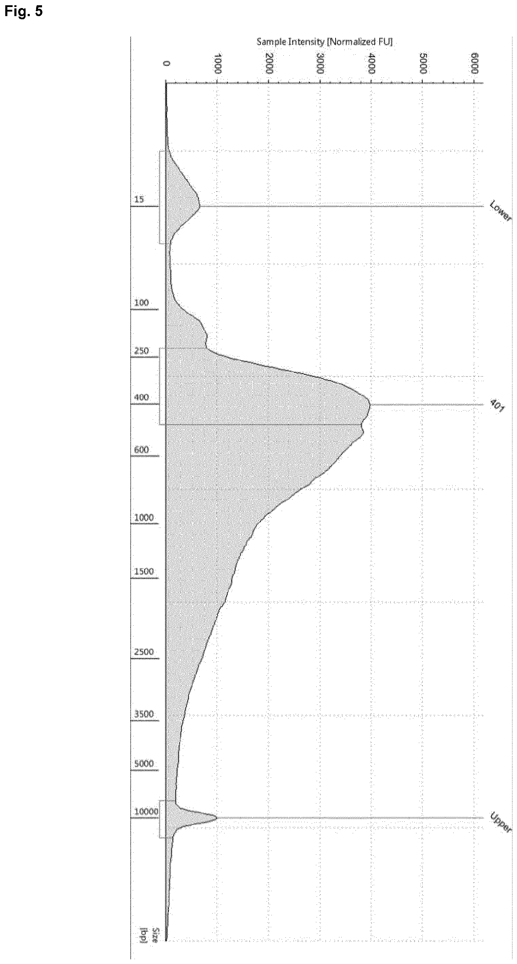

**Figure 5****:** Chromatogram showing the size distribution of a final sequencing library derived from 3 pooled single cell reactions (Step 6). The Library exhibits a main peak at around 400 bp. The majority of library amplicons is smaller than 1000 bp.

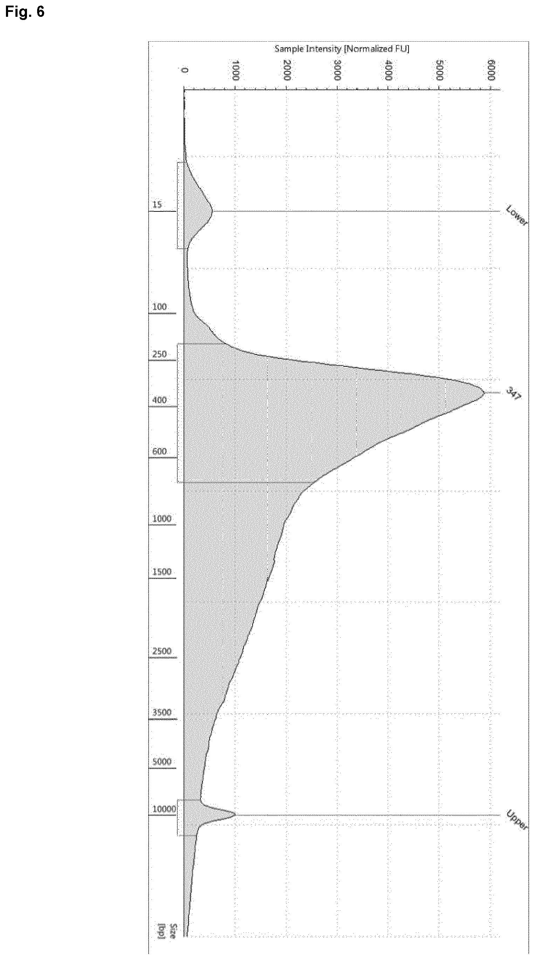

**Figure 6****:** Chromatogram showing the size distribution of a final sequencing library derived from a single reaction (positive control) with 30 pg gDNA (Step 6). The Library exhibits a main peak at around 350 bp. The majority of library amplicons is smaller than 1000 bp.

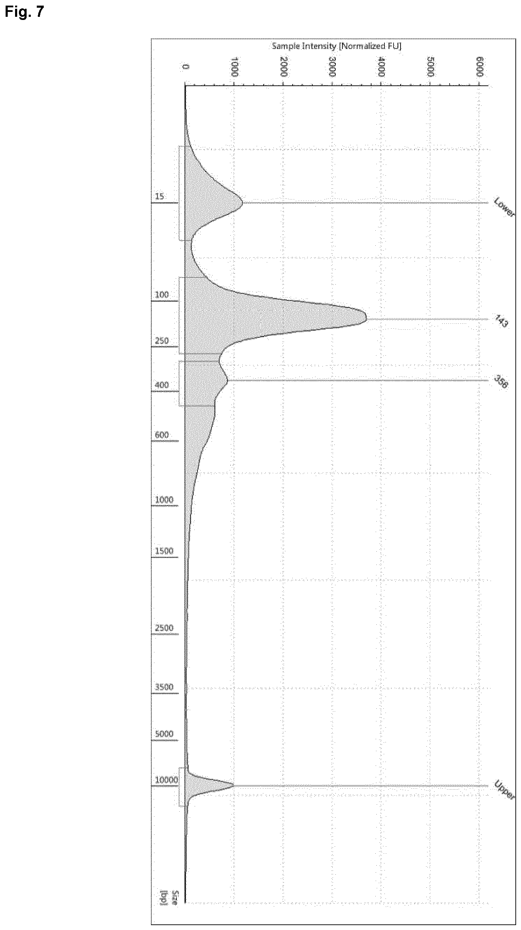

**Figure 7****:** Chromatogram showing the size distribution of adapter dimers and template independent products from a single reaction without template (negative control).

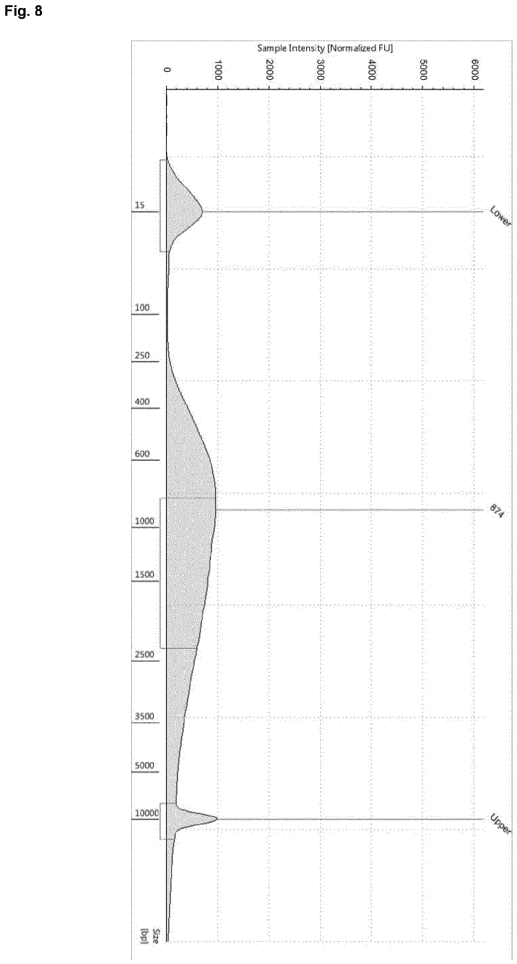

**Figure 8****:** Chromatogram showing the size distribution of amplicons derived from a single reaction (1 single cell) with regular MALBAC PCR performed after quasilinear amplification (Step 4). The Library exhibits a main peak at around 880 bp. A large portion of library amplicons are larger than 1000 bp.

### EXAMPLES

The invention is further described by the following examples. These are not intended to limit the scope of the invention but represent preferred embodiments of aspects of the invention provided for greater illustration of the invention described herein.

### Reagents

- CellsDirect Resuspension & Lysis Buffers; Thermo; 11739010
- QIAGEN Protease (7.5 AU); Qiagen; 19155
- ThermoPol® II (Mg-free) Reaction Buffer; NEB; B9005
- Deoxynucleotide (dNTP) Solution Mix; NEB; N0447S
- Magnesium Sulfate (MgSO4) Solution: NEB; B1003S
- 5x Q-Solution; Qiagen
- Deep Vent® (exo-) DNA Polymerase; NEB; M0259S
- SD Polymerase, 10 U / µl; Bioron; 108702
- BSA, Molecular Biology Grade; NEB; B9000S
- Lambda Exonuclease; NEB; M0262S
- β-Nicotinamide adenine dinucleotide (NAD+); NEB; B9007S
- Ampligase® Enzyme and Buffer; Lucigen; A32750
- *Taq* DNA Polymerase; NEB; M0273S
- Q5® Hot Start High-Fidelity DNA Polymerase; NEB; M0493S
- DNA LoBind Tubes, DNA LoBind, 1,5 mL, PCR clean; Eppendorf; 0030108051
- Pooling Beads:
   ∘ 30% w/v PEG 8000
   ∘ 2M NaCl
   ∘ 10 mM Tris-HCI pH 8.0
   ∘ 2 mM EDTA
   ∘ 0.01 % IGEPAL
   ∘ 0.05 % Sodium Acide
   ∘ 0.05% tween 20
   ∘ 2µl / ml Sera-Mag SpeedBead Carboxylate-Modified Magnetic Particles (Hydrophobic), 15 mL; GE; 65152105050250

### Oligonucleotides

*Primer A:*
P-ATGGAGAGTGATTGGAGAGGTAGTGCGAAGD*D*D*D*DNNNNNNN (SEQ ID NO1)
- HPLC purified oligonucleotide
- P = 5' Phosphate
- D = single-cell barcode, 5 bases (G, A and T)
- * = phosphorothioate internucleotide linkages
- N = random nucleotides

**Table 1. Single-cell barcode sequences of primer A:**

| BC# | sequence | SEQ ID NO |
|---|---|---|
| 1 | aaaga | SEQ ID NO11 |
| 2 | aaatg | SEQ ID NO12 |
| 3 | aagaa | SEQ ID NO13 |
| 4 | aatgt | SEQ ID NO14 |
| 5 | agata | SEQ ID NO15 |
| 6 | agtat | SEQ ID N016 |
| 7 | ataag | SEQ ID NO17 |
| 8 | atagt | SEQ ID NO18 |
| 9 | atgat | SEQ ID NO19 |
| 10 | attga | SEQ ID NO20 |
| 11 | atttg | SEQ ID NO21 |
| 12 | gattt | SEQ ID NO22 |
| 13 | gtaat | SEQ ID NO23 |
| 14 | gtata | SEQ ID NO24 |
| 15 | gttaa | SEQ ID NO25 |
| 16 | taaag | SEQ ID NO26 |
| 17 | taagt | SEQ ID NO27 |
| 18 | tagat | SEQ ID NO28 |
| 19 | tatga | SEQ ID NO29 |
| 20 | tattg | SEQ ID NO30 |
| 21 | tgaat | SEQ ID NO31 |
| 22 | tgtta | SEQ ID NO32 |
| 23 | ttaga | SEQ ID NO33 |
| 24 | ttatg | SEQ ID NO34 |

*Primer B:*
P-ATGGAGAGTGATTGGAGAGGTAGTGCG*A*A*G (SEQ ID NO2)
- HPLC purified oligonucleotide
- P = 5' Phosphate
- * = phosphorothioate internucleotide linkages
*Primer C:*
AATGATACGGCGACCACCGAGATCTACACTCTTTCCCTACAGAGAGGTAGTGC (SEQ ID NO3)
- HPLC or PAGE purified oligonucleotide
- Underlined: Adapter sequence Illumina P5
*Primer D:* 
CAAGCAGAAGACGGCATACGAGATNNNNNNCGAATGGAGAGTGATTGGAGAGGTAGTGCG
AAG
(SEQ ID NO7)
- HPLC or PAGE purified oligonucleotide
- N = Illumina 7 bp barcode
- Underlined: Adapter sequence Illumina P7

### Step 1: Single cell deposition

1. Perform single-cell isolation using an appropriate method (e.g. single-cell printing or FACS).
2. Store on ice until use or freeze immediately at -80°C.

### Step 2: Cell Lysis and Chromatin Digestion

1. Prepare sufficient cell lysis buffer: Mix Resuspension Solution and Lysis Enhancer in a 10:1 ratio (e.g. 150 µl Resuspension Solution + 15 µl Lysis Enhancer; sufficient for 96 reactions).
2. Carefully dispense 1.5 µl of lysis buffer to each well of the 96 well plate containing a single cell.
3. For positive controls use 30 pg gDNA per reaction diluted in lysis buffer
4. For negative controls do not dispense a cell.
5. Spin down the plate at 2000 x g and 4 °C for 10 sec.
6. Incubate at 75°C for 15 min in a thermal cycler with the lid heated to 85°C.
7. Carefully dispense 0.5 µl of Qiagen Protease (1.875 AU/ml) to each reaction.
8. Spin down the plate at 2000 x g and 4 °C for 10 sec.
9. Incubate at 50°C for 90 and at 75°C for 30 min in a thermal cycler with the lid heated to 85°C.

### Step 3: Quasilinear Amplification

1. Dispense 0.5 µl of a specific barcoded PRIMER A (10 µM) to each well of the 96 well plate containing a single cell.
2. Spin down at 2000 x g and 4 °C for 10 sec.
3. Set up the quasilinear amplification master mix per reaction as follows (on ice):

| | |
|---|---|
| Thermo Pol Buffer Mg free | 1 |
| dNTP Mix 10 mM | 0,2 |
| Mg Solution (100 mM) | 0,35 |
| Betaine 5M (Qiagen Q Solution) | 2 |
| Deep Vent® (exo-) DNA Polymerase | 0,25 |
| Bioron SD Polymerase | 0,02 |
| H₂O | 3,68 |
| Total | 7.5 |

4. Carefully dispense 7.5 µl of the master mix to each single cell reaction.
5. Spin down at 2000 x g and 4 °C for 10 sec.
6. Mix on a plate shaker at 800 rpm for 1 min.
7. Spin down at 2000 x g and 4 °C for 10 sec.
8. Run the following program on a thermal cycler:

| |
|---|
| 1: 10°C 2 min; ramp between steps 0.3°C/sec |
| 2: 20°C 50 sec; ramp between steps 0.3°C/sec |
| 3: 30°C 45 sec; ramp between steps 0.3°C/sec |
| 4: 40°C 45 sec; ramp between steps 0.3°C/sec |
| 5: 50°C 45 sec; ramp between steps 3.0°C/sec |
| 6: 70°C 2 min; ramp between steps 3.0°C/sec |
| 7: 92°C 30 sec; ramp between steps 3.0°C/sec |
| 8: 66°C 20sec |
| go to 2 12X |
| 9: 4°C forever |

### Step 4 (optional): regular MALBAC PCR after Quasilinear Amplification

1. Set up the MALBAC master mix per reaction as follows (on ice):

| | |
|---|---|
| Thermo Pol Buffer Mg free | 1,5 |
| Primer B (10 µM) | 1,2 |
| dNTP Mix 10 mM | 0,7 |
| Deep Vent® (exo-) DNA Polymerase | 0,19 |
| Mg Solution (100 mM) | 0,42 |
| H₂O | 10.99 |
| Total | 15 µl |

2. Carefully transfer the single cell reaction to a fresh 200 µl PCR tube and mix with 15 µl of the master mix.
3. Briefly spin down
4. Run the following program on a thermal cycler:

| |
|---|
| 1: 95°C 1 min |
| 2: 95°C 20 sec |
| 3: 60°C 30 sec |
| 4: 72°C 4 min |
| go to 2 20X |
| 5: 4°C forever |

### Step 5: Conversion to double stranded DNA

1. Set up the dsDNA master mix per reaction as follows (on ice):

| | |
|---|---|
| Thermo Pol Buffer Mg free | 0,5 |
| Primer B (10 µM) | 1,5 |
| dNTP Mix 10 mM | 0,1 |
| Mg Solution (100 mM) | 0,07 |
| H₂O | 2,83 |
| Total | 5 µl |

2. Carefully dispense 5 µl of the master mix to each quasilinear amplification reaction (Step 3).
3. Spin down the plate at 2000 x g and 4 °C for 10 sec.
4. Mix on a plate shaker at 800 rpm for 1 min.
5. Spin down the plate at 2000 x g and 4 °C for 10 sec.
6. Run the following program on a thermal cycler:

| |
|---|
| 1: 95°C 1 min |
| 2: 66°C 30 sec |
| 3: 72°C 4 min |
| 4: 85°C 1 min |
| 5: 66°C 30 sec |
| 6: 72°C 4 min |
| go to 4 6X |
| 7: 4°C forever |

Optional pause point: The dsDNA amplicons can be stored at -20 °C over night.

### Step 6: Pooling and Purification of single cell reactions

1. Transfer and pool all single cell reactions (each 15µl) in a 1.5 ml DNA low binding tube using the same pipette tip. Up to 24 single-cell reactions can be pooled in one 1.5 ml DNA low binding tube.
2. Wash each empty well with 15 µl 0.5x TE and transfer to the pooled single cell reactions in order to minimize loss of DNA.
3. Add pooling beads in a 1:1.1 ratio to the pooled single cell reactions (e.g. per reaction 15µl single cell reaction + 15µl 0.5x TE + 33 µl pooling beads). Mix carefully but thoroughly by Pipetting.
4. Spin down briefly and incubate on horizontal shaker (e.g. Eppendorf thermomixer) at 800 rpm for 25 min.
5. Place the tube against an appropriate magnetic stand for at least 10 min to separate the beads from the supernatant (the supernatant should be clear). Remove the supernatant.
6. Add 200 µl of 80% freshly prepared ethanol to the tube while in the magnetic stand and incubate at room temperature for 30 seconds. Carefully remove and discard the supernatant. Do not disturb the beads that contain DNA targets.
7. Repeat the ethanol wash once for a total of two washes. Remove all visible liquid after the second wash using a p10 pipette tip.
8. Air dry the beads for approximately 2 minutes while the tubes are on the magnetic stand with the lid open. Do not overdry the beads!
9. Remove the tubes from the magnetic stand and Elute the DNA target from the beads with 8 µl of NEB Elution Buffer (10 mM Tris, 0.1 mM EDTA, pH 8.5) and transfer to a fresh 200 µl PCR tube.

### Step 7: Exonuclease Treatment

1. Set up the exonuclease master mix per reaction as follows (on ice):

| | |
|---|---|
| Thermo Pol Buffer Mg free | 0,9 |
| Mg Solution (100 mM) | 0,225 |
| BSA 10 mg/ml | 0,045 |
| Lambda exonuclease | 0,5 |
| H₂O | 0,33 |
| Total | 2 |

2. Carefully dispense 2 µl of the master mix to each 8µl of pooled single cell amplicons
3. Mix on a plate shaker at 800 rpm for 1 min.
4. Spin down the plate at 2000 x g and 4 °C for 10 sec.
5. Run the following program on a thermal cycler:

| |
|---|
| 1: 37°C 30 min |
| 2: 75°C 10 min |
| 3: 4°C forever |

### Step 8: Gap-filling / Ligation

1. Set up the gap-filling / ligation master mix per reaction as follows (on ice):

| | |
|---|---|
| Thermo Pol Buffer Mg free | 0,35 |
| Mg Solution (100 mM) | 0,15 |
| NAD Solution (50 mM) | 0,125 |
| Ampligase | 0,5 |
| Primer C (50 µM) | 1 |
| dNTP Mix 10 mM | 0,25 |
| Taq Pol | 0,05 |
| H₂O | 1,075 |
| Total | 3,5 |

2. Carefully dispense 3.5 µl of the master mix to each reaction.
3. Spin down the plate at 2000 x g and 4 °C for 10 sec.
4. Mix on a plate shaker at 800 rpm for 1 min.
5. Spin down the plate at 2000 x g and 4 °C for 10 sec.
6. Run the following program on a thermal cycler:

| |
|---|
| 1: RAMP: 70°C-37°C (0,1°C/sec) |
| 2. 37°C 30 min |
| 3: 4°C forever |

### Step 8: Sequencing Library PCR

1. Set up the PCR master mix per reaction as follows (on ice):

| | |
|---|---|
| Q5 Buffer | 7,5 |
| dNTP Mix 10 mM | 0,75 |
| Primer D (10 µM) | 5 |
| Q5 Hot Start Polymerase | 0,5 |
| H₂O | 22,75 |
| | 36,5 |

2. Carefully dispense 36.5 µl of the master mix to each reaction.
3. Spin down the plate at 2000 x g and 4 °C for 10 sec.
4. Mix on a plate shaker at 800 rpm for 1 min.
5. Spin down the plate at 2000 x g and 4 °C for 10 sec.
6. Run the following program on a thermal cycler:

| |
|---|
| 1: 98°C 30 sec |
| 2: 98°C 10 sec |
| 3: 70°C 15 sec |
| 4: 65°C 1 min |
| go to 2, 15-20X* |
| 6: 72°C 4 min |
| 7: 4°C forever |

## Claims

1. **A method of amplifying DNA of more than one samples** for generating a sequencing library, the method comprising
a. providing for each of the more than one samples an individual reaction vessel comprising the DNA in single stranded form,
b. adding first primers to the reaction vessel to produce a reaction mixture,
wherein the first primers comprise in a 5' to 3' orientation a constant sequence and a variable sequence,
wherein the constant sequence comprises in a 5' to 3' orientation a first part with a sequence that can be common for the more than one samples and a second part with a sequence that is different for each of the more than one samples,
c. adding a DNA polymerase to the reaction vessel,
d. subjecting the reaction mixture to a temperature at which the first primers anneal to the single stranded DNA via the variable sequence,
e. subjecting the reaction mixture to a temperature at which the DNA polymerase extends the 3'-ends of the annealed first primers to produce first-round amplicons,
f. subjecting the reaction mixture to a temperature to produce single stranded amplicons,
g. subjecting the reaction mixture to a temperature at which free first primers anneal to the provided single stranded DNA and to the first-round amplicons via the variable sequence,
h. subjecting the reaction mixture to a temperature at which the DNA polymerase extends the 3'-ends of the annealed first primers to produce first-round amplicons from first primers annealed to the provided DNA and second-round amplicons from first primers annealed to first-round amplicons,
i. subjecting the reaction mixture to a temperature to produce single stranded amplicons,
j. repeating the steps (g)-(i) to produce second-round amplicons of the provided DNA.

2. The method according to any of the preceding claims, wherein between step (i) and (j) the reaction mixture is subjected to a temperature at which second-round amplicons form a hairpin structure through hybridization of the complementary constant sequences at the 5' and 3' end of the second-round amplicons.

3. The method according to any of the preceding claims, comprising additionally
k. adding to the reaction mixture of step (j) second primers comprising at their 3'-end a sequence comprised by the first part of the constant sequence of the first primers,
I. subjecting the reaction mixture to a temperature to produce single stranded amplicons,
m. subjecting the reaction mixture to a temperature at which the second primers anneal to the first part of the constant sequence of the second-round amplicons,
n. subjecting the reaction mixture to a temperature at which the DNA polymerase extends the 3'-ends of the annealed second primers to produce double stranded third-round amplicons,
o. subjecting the reaction mixture to a temperature to produce single stranded amplicons,
p. repeating the steps (m)-(o) to produce double stranded third-round amplicons.

4. The method according to the preceding claim, wherein the temperature to produce single stranded amplicons of step (o) is a temperature at which hairpin structures formed by second-round amplicons dehybridize and double stranded third-round amplicons do not dehybridize.

5. The method according to any of the preceding claims, wherein after step (j) or (p) the reaction mixtures of the more than one samples are pooled in a single reaction vessel, and wherein the sequence of the first part of the constant sequence is common for all of the more than one samples.

6. The method according to any of the preceding claims, wherein the more than one samples are more than one single cells and/or wherein the DNA is genomic DNA.

7. The method according to any of the preceding claims, wherein the variable sequence of the first primers comprises between 3 and 12 nucleotides, which are randomly selected from among G, T, A and C, wherein the variable sequence is configured to enable the first primers to anneal randomly to single-stranded DNA molecules, preferably single-stranded genomic DNA molecules.

8. The method according to any of the preceding claims, wherein the constant sequence of the first primers comprises between 10 and 70 nucleotides, preferably about 10-40 nucleotides, wherein the first part of the constant sequence comprises between 5 and 66 nucleotides, preferably 5-36 nucleotides and the second part of the constant sequence comprises between 4 and 10 nucleotides.

9. The method according to any of claims 3 to 8, wherein the second primers consist of a sequence comprised by the first part of the constant sequence of the first primers, wherein the second primers preferably consist of 10-66 nucleotides, more preferably of 20-40 nucleotides, most preferably of about 25-35 nucleotides.

10. The method according to any one of the preceding claims, wherein the amplicons have an average length off less than 1000 nucleotides.

11. The method according to any of the preceding claims, wherein the first primers comprise in the constant sequence, preferably at the 3'-end of the first part of the constant sequence and/or in the second part of the constant sequence, preferably at the 5'-end of the second part of the constant sequence, and/or the second primers comprise in the sequence at their 3'-end, preferably at their 3'-end, modified nucleotides resistant to a 5'-exonuclease, such as nucleotides with a phosphorothioate modification or LNA nucleotides, wherein preferably all nucleotides of the second part of the constant sequence of the first primers are modified and/or 3 to 6 nucleotides comprised by the sequence at the 3'-end of the second primers are modified, and/or
wherein the first and second primers are phosphorylated at their 5'-ends.

12. **A method of generating a sequencing library** comprising the steps of
a. providing in a reaction vessel double-stranded DNAs (dsDNAs), such as dsDNA fragments or dsDNA amplicons, wherein preferably each strand of the dsDNAs comprises near its 5'-end, preferably within nucleotides 20-100, more preferably within the nucleotides 25-40 counted from the 5'-end, at least one modified nucleotide resistant to a 5'-exonuclease, such as nucleotides with a phosphorothioate modification or LNA nucleotides,
b. adding a 5'-exonuclease to the reaction vessel to produce a reaction mixture,
c. subjecting the reaction mixture to a temperature at which the 5'-exonuclease is active to generate dsDNA with overhanging single-stranded 3'-ends,
d. adding to the reaction vessel
- gap-filling primers, wherein the gap-filling primers comprise
∘ at their 3'-end a sequence that enables annealing of the gap-filling primers to the overhang-sequences at the 3'-ends of the dsDNA, and
∘ a first adapter sequence for a subsequent sequencing of the dsDNA,
- a DNA polymerase, and
- a ligase,
e. subjecting the reaction mixture to a temperature at which
- a gap-filling primer hybridizes to the overhanging single-stranded 3'-end of a dsDNA,
- the 3'-end of the third primer is extended by the DNA polymerase and
- the extended 3'-end of the gap-filling primer is ligated to a 5'-end of a strand of the dsDNA by the ligase.

13. The method of generating a sequencing library according to the preceding claim, comprising after step (e)
f. adding adapter primers to the reaction vessel, wherein the adapter primers comprise
- at their 3'-end a sequence that enables hybridization/annealing of the adapter primers to the overhang-sequences at the 3'-ends of the dsDNA with a higher affinity than the sequence at the 3'-end of the gap-filling primers, and
- a second adapter sequence for a subsequent sequencing of the dsDNA,
g. subjecting the reaction mixture to a temperature to produce single stranded DNA (ssDNA),
h. subjecting the reaction mixture to a temperature at which the adapter primers anneal to the 3'-ends of the ssDNA comprising ligated gap-filling primers at their 5'-end,
i. subjecting the reaction mixture to a temperature at which the DNA polymerase extends the 3'-ends of the annealed adapter primers and of the ssDNA comprising ligated gap-filling primers to produce double-stranded adapter amplicons.

14. The method of generating a sequencing library according to the preceding claim, comprising after step (i)
j. subjecting the reaction mixture to a temperature to produce single stranded adapter amplicons,
k. subjecting the reaction mixture to a temperature at which the adapter primers and gap-filling primers anneal to the 3'-ends of the corresponding single-stranded adapter amplicons,
I. subjecting the reaction mixture to a temperature at which the DNA polymerase extends the 3'-ends of the annealed adapter and gap-filling primers to produce double stranded adapter amplicons,
m. repeating the steps (j) - (I) to produce double stranded adapter amplicons.

15. The method according to any of claims 12-14, wherein the provided dsDNAs are third-round amplicons generated by the method of amplifying DNA according any of claims 3-11.

16. **A kit for amplifying DNA of more than one samples** for generating a sequencing library, the kit comprising at least two kinds of first primers,
- wherein each kind of first primers is provided in an individual container,
- wherein each kind of first primer comprises in a 5' to 3' orientation a constant sequence and a variable sequence,
- wherein the constant sequence comprises in a 5' to 3' orientation a first part with a sequence that is common for each of the at least two kinds of first primers, and a second part with a sequence that is different for each of the at least two kinds of first primers.

17. The kit according to claim 16, comprising at least 12, preferably at least 96, more preferably 384 kinds of first primers, wherein the primers are preferably provided in sealed individual wells or a multiwell plate.

18. The kit according to any one of claims 16 or 17, wherein
- each kind of first primers comprises in the constant sequence modified nucleotides resistant to a 5'-exonuclease, such as nucleotides with a phosphorothioate modification or LNA nucleotides, wherein preferably at least 3 to 6 nucleotides of the second part of the constant region are modified, wherein the modified nucleotides are preferably located at the 5'-end of the second part of the constant region,
- and/or wherein the first primers are phosphorylated at their 5'-ends,
- and/or wherein each kind of first primers comprises between 10 and 70 nucleotides, preferably about 10-40 nucleotides, wherein the first part of the constant sequence comprises between 5 and 66 nucleotides, preferably 5-36 nucleotides and the second part of the constant sequence comprises between 4 and 10 nucleotides.

19. The kit according to any one of claims 16 to 18, comprising additionally in an individual container
- second primers comprising
at their 3'-end a sequence comprised by the first part of the constant sequence of the first primers and
at their 3'-end at least one modified nucleotide, preferably at least 3 to 6 modified nucleotides, resistant to a 5'-exonuclease, such as nucleotides with a phosphorothioate modification or LNA nucleotides,
wherein the second primers are preferably phosphorylated at their 5'-ends, and optionally
- gap-filling primers comprising
at their 3'-end a sequence comprised by the sequence at the 3'-end of the second primers, wherein the sequence at the 3'-end of the gap-filling primers enables annealing of the gap-filling primers to the sequence at the 3'-end of the second primers, and
a first adapter sequence for a subsequent sequencing of the amplicons,
and optionally
- adapter primers comprising
at their 3'-end a sequence comprised by the sequence at the 3'-end of the second primers, wherein the sequence at the 3'-end of the adapter primers enables annealing of the adapter primers to the sequence at the 3'-end of the second primers, preferably with a higher affinity than the sequence at the 3'-end of the gap-filling primers, and
a second adapter sequence for a subsequent sequencing of the amplicons,
- and optionally one or more enzymes for performing any of the methods according to claims 1 to 15.


SEQUENCE LISTING

<110> Albert-Ludwigs-Universität Freiburg

<120> METHODS FOR AMPLIFICATION OF GENOMIC DNA AND
PREPARATION OF SEQUENCING LIBRARIES

<130> 2198/19EP

<160> 34

<170> BiSSAP 1.3.6

<210> 1
<211> 42
<212> DNA
<213> Artificial Sequence

<220>
<223> First Primer #1 (Primer A)

<400> 1
atggagagtg attggagagg tagtgcgaag dddddnnnnn nn                42

<210> 2
<211> 30
<212> DNA
<213> Artificial Sequence

<220>
<223> Second Primer #1 (Primer B)

<400> 2
atggagagtg attggagagg tagtgcgaag                              30

<210> 3
<211> 53
<212> DNA
<213> Artificial Sequence

<220>
<223> Gap-filling Primer #1 (Primer C)

<400> 3
aatgatacgg cgaccaccga gatctacact ctttccctac agagaggtag tgc    53

<210> 4
<211> 12
<212> DNA
<213> Artificial Sequence

<220>
<223> Sequence at the 3-end of the gap-filling primer

<400> 4

gagaggtagt gc                                                          12

<210> 5
<211> 20
<212> DNA
<213> Artificial Sequence

<220>
<223> Illumina P5 Adapter sequence

<400> 5
aatgatacgg cgaccaccga                                                  20

<210> 6
<211> 53
<212> DNA
<213> Artificial Sequence

<220>
<223> Gap-filling Primer #2

<400> 6
aatgatacgg cgaccaccga tagctcacat ctttccctac agagaggtag tgc             53

<210> 7
<211> 63
<212> DNA
<213> Artificial Sequence

<220>
<223> Adapter Primer #1 (Primer D)

<400> 7
caagcagaag acggcatacg agatnnnnnn cgaatggaga gtgattggag aggtagtgcg      60

aag                                                                    63

<210> 8
<211> 30
<212> DNA
<213> Artificial Sequence

<220>
<223> Sequence at the 3-end of the adapter primer

<400> 8
atggagagtg attggagagg tagtgcgaag                                       30

<210> 9
<211> 24
<212> DNA
<213> Artificial Sequence

<220>
<223> Illumina P7 Adapter sequence (without index)

<400> 9
caagcagaag acggcatacg agat                                              24


<210> 10
<211> 63
<212> DNA
<213> Artificial Sequence


<220>
<223> Adapter Primer #2

<400> 10
caagcagaag acggcatacg agatnnnnnn cctatggaga gtgattggag aggtagtgcg      60

aag                                                                     63


<210> 11

<400> 11

000


<210> 12

<400> 12

000


<210> 13

<400> 13

000


<210> 14

<400> 14

000


<210> 15

<400> 15

000


<210> 16

<400> 16

000

<210> 17

<400> 17

000

<210> 18

<400> 18

000

<210> 19

<400> 19

000

<210> 20

<400> 20

000

<210> 21

<400> 21

000

<210> 23

<400> 23

000

<210> 24

<400> 24

000

<210> 25

<400> 25

000

<210> 26

<400> 26

000

<210> 27

<400> 27

000

<210> 28

<400> 28

000

<210> 29

<400> 29

000

<210> 30

<400> 30

000

<210> 31

<400> 31

000

<210> 32

<400> 32

000

<210> 33

<400> 33

000

<210> 34

<400> 34

000